# EUROPEAN PATENT APPLICATION

(11) **EP 2 878 677 A1**
(43) Date of publication of application: **03.06.2015**
(21) Application number: 13194865.5
(22) Date of filing: 28.11.2013
(51) Int. Cl.: C12Q 1/42

(54) **Activators of protein phosphatase 5**

(71) Applicant: Deutsches Zentrum für Neurodegenerative Erkrankungen e.V. (DZNE), 53175 Bonn (DE); Technische Universität München, 80333 München (DE)
(72) Inventor: Groll, Michael, 80333 Munich (DE); Richter, Klaus, 80637 Munich (DE); Haslbeck, Veronika, 88400 Biberach/Riss (DE); Striggow, Frank, 39175 Gerwisch (DE); Martin, Helmuth, 39118 Magdeburg (DE); Schmidt, Werner, 17268 Boitzenburger Land (DE); Weiwad, Matthias, 06120 Halle (Saale) (DE); Fischer, Gunter, 06120 Halle (DE)
(74) Representative: Engelhard, Markus

(57) **Abstract**

The present invention relates to compounds that show a positive result in a phosphatase activation assay with protein phosphatase 5 according to the invention, to compounds that bind in an *in vitro* binding assay to a certain region of the amino acid sequence of protein phosphatase 5, as well as to compounds that show a positive result in a phosphatase activation assay with protein phosphatase 5 according to the invention and bind in an *in vitro* binding assay to a certain region of the amino acid sequence of protein phosphatase 5. The present invention also relates to pharmaceutical compositions comprising one or more of such compounds, to methods of using such a compound, and to methods of screening for compounds that activate protein phosphatase 5.

## Description

The present invention relates to compounds that show a positive result in a phosphatase activation assay with protein phosphatase 5 according to the invention, to compounds that bind in an *in vitro* binding assay to a certain region of the amino acid sequence of protein phosphatase 5, as well as to compounds that show a positive result in a phosphatase activation assay with protein phosphatase 5 according to the invention and bind in an *in vitro* binding assay to a certain region of the amino acid sequence of protein phosphatase 5. The present invention also relates to pharmaceutical compositions comprising one or more of such compounds, to methods of using such a compound, and to methods of screening for compounds that activate protein phosphatase 5.

Protein phosphatases are functional counterparts of kinases. Collectively, both act as molecular check points within a wide spectrum of signaling cascades. Protein phosphatase 5 (PP5) belongs to a handful of intracellular protein phosphatases. It shares a high degree of homology within the phosphatase domain to PP1, PP2A and PP2B/PP3, all members of the phosphoprotein phosphatase (PPP) family. In contrast to the latter phosphatases, PP5 contains an N-terminal tetratricopeptide repeat (TPR) domain and a C-terminal αJ subdomain. The contact between those two locks the enzyme in an autoinhibited state (Chen et al., 1994; Yang et al., 2005).

While other PPP family members are modulated by the interaction of various subunits to control catalytic activity and substrate specificity of the holoenzyme, PP5 is governed by its autoinhibitory domains encoded on the same polypeptide. In contrast to other PPP family members that are stimulated by accessory subunits, PP5 is an Hsp90-regulated enzyme (Ramsey and Chinkers, 2002; Yang et al., 2005). In the absence of the molecular chaperone Hsp90, PP5 exhibits only low basal activity. The C-terminal helix of PP5, the so-called αJ subdomain, binds the N-terminal TPR domain of PP5 and locks the enzyme in a latent state. In this conformation, residues of the TPR domain and αJ helix are positioned in a way that limits substrate entry to the active site of the phosphatase core domain (Yang et al., 2005). PP5 forms high affinity complexes with Hsp90. In particular, the C-terminal MEEVD-peptide of Hsp90 is recognized by the phosphatase's TPR domain with low micromolar affinity and thereby, stabilizes the structure of the TPR domain of PP5 (Cliff et al., 2006). Hsp90-PP5 complexes have also been described in yeast and nematodes. This interaction consequently causes the release of the intramolecular contacts between TPR and αJ domain of autoinhibited PP5 and opens the active site access. Upon complex formation with Hsp90, the phosphatase activity of PP5 is fully activated and thus, diverse Hsp90-interacting proteins, e.g. Cdc37, can be dephosphorylated with increased rates.

Cdc37 is a kinase-dedicated Hsp90 co-chaperone that delivers immature kinases to the Hsp90 chaperone machinery. Its dephosphorylation by PP5 represents a critical step within the activation of kinase clients of Hsp90.

It has been suggested that Hsp90 may enhance the phosphatase activity of PP5 also towards Hsp90-attached phosphate residues. This mode of action permits a PP5-dependent control of the Hsp90-chaperone machinery.

PP5 is localized in the cytosol and nucleus and is predominantly found in the brain(Bahl et al., 2001; Chen et al., 1994). This protein is known to be involved in various cellular processes, ranging from steroid hormone receptor regulation, cell cycle control to neuronal degeneration. Early studies have identified PP5 as part of Hsp90-glucocorticoid receptor (GR) complexes. Later, PP5 was shown to affect the translocation of the hormone-activated complex into the nucleus and to directly dephosphorylate GR complexes. After DNA-damage or during cell cycle control, PP5 is able to dephosphorylate the kinases ASK1 and DNA-PKcs. In addition, PP5 is associated with the G1/S-phase checkpoint regulators ATM and ATR.

Hyperphosphorylation of the human microtubule-associated protein tau has been suggested to be crucial in the pathogenesis of tauopathies such as Alzheimers's disease (AD) and frontotemporal lobar degeneration, including dementia with parkinsonism linked to chromosome 17 (FTDP-17). Tau is phosphorylated by a variety of protein kinases, e.g. glycogen synthase kinase (GSK) 3β, mitogen-activated protein kinase (MAPK) 1, cyclin-dependent kinase (Cdk) 5, casein kinase and MAP/microtubule affinity-regulating kinase (MARK) 1 and tau hyperphosphorylation promotes its dissociation from axonal microtubules (Brunden et al., 2009). In addition, tau phosphorylation, e.g. at the AT8 (Ser202/Thr205) and PHF1 (Ser396/Ser404) epitopes, triggers conformational changes from its intrinsically disordered towards a pathology-relevant, misfolded state (Jeganathan et al., 2008). These structural rearrangements promote tau aggregation and the formation of neurofibrillar tangles (NFTs) in the somatodendritic compartment (Brunden et al., 2009; Li et al., 2011).

NFTs are, besides extracellular amyloid ß plaques, another hallmark of AD. Several previous studies have revealed tau-dependent defects in synaptic plasticity and learning and memory. According to the amyloid cascade hypothesis, tau is required to mediate the neurotoxic effects of amyloid ß. Nevertheless, the molecular mechanism linking amyloid ß and its executor tau remains unclear (Haass, 2010; Jack et al., 2013).

Hyperphosphorylated tau can be dephosphorylated by PP5 and other PPP family members (Liu et al., 2005a; Liu et al., 2005b). PP5 is highly expressed in the mammalian brain, but few physiological substrates have yet been identified (Liu et al., 2005b). Notably, activity levels of PP5 have been found to be reduced in AD brains (Liu et al., 2005a). These findings imply that enhanced phosphatase activities, including that of PP5, may cause beneficial effects in AD and other human tauopathies.

Despite the important cellular functions of protein phosphatases, only a few small molecule effectors which are highly selective for certain phosphoprotein phosphatase (PPP) family members have been identified yet (McConnell and Wadzinski, 2009). Arachidonic acid and its derivatives have been found to stimulate PP5, but the physiological relevance remains elusive (Cher et al., 2010; Kang et al., 2001; Ramsey and Chinkers, 2002). Efforts to analyze the underlying mode of activation have suggested a crucial involvement of the PP5 TPR domain which releases the autoinhibition of the enzyme upon binding of arachidonic acid (Cher et al., 2010). Contrarily, synthetic small molecule activators that target PP5 have not been identified so far.

Thus, there is a need in the art for protein phosphatase 5 activators, in particular small molecule activators, that are synthetic and/or have improved characteristics. Moreover, there is a need in the art for methods to identify such activators.

It is therefore an object of the present invention to provide for activators, in particular small molecule activators, that target protein phosphatase 5. Moreover, it is an object of the present invention to provide for small molecule activators of protein phosphatase 5 with improved characteristics. Thus, it is for example an object of the present invention to provide for small molecule activators of protein phosphatase 5 that have a higher water solubility compared to protein phosphatase 5 small molecule activators known in the art, that, preferably, have nevertheless good membrane diffusion characteristics, that are suitable for administration to a patient via the oral route, and/or that show improved specificity. Furthermore, it is an object of the present invention to provide for small molecule activators of protein phosphatase 5 that do not affect the direct interaction of protein phosphatase 5 with Hsp90. Moreover, it is an object of the present invention to provide for small molecule activators of protein phosphatase 5 that show fewer or less severe side effects if used in a patient. Furthermore, it is an object of the present invention to provide for small molecule activators of protein phosphatase 5 that are synthetic and do not occur in nature. Moreover, it is an object of the present invention to provide for methods to identify activators, in particular small molecule activators, of protein phosphatase 5. Furthermore, it is an object of the present invention to provide for simple and straight-forward screening methods that allow to identify small molecule activators as described herein. In addition, it is an object of the present invention to provide for novel treatment alternatives for the treatment of tauopathies or cancer.

The objects of the present invention are solved by a compound that shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
and wherein a result is considered a positive result if the enzymatic activity of protein phosphatase 5 in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested.

The objects of the present invention are also solved by a compound that binds to protein phosphatase 5 within the region of amino acids 166 to 496 of the C. *elegans* protein phosphatase 5 amino acid sequence or within the region of amino acids 166 to 499 of the mammalian protein phosphatase 5 amino acid sequence or within the region of amino acids 166 to 499 of the rat protein phosphatase 5 amino acid sequence or within the region of amino acids 166 to 499 of the mouse protein phosphatase 5 amino acid sequence or within the region of amino acids 166 to 499 of the human protein phosphatase 5 amino acid sequence. Preferably, said binding is determined in an *in vitro* binding assay, more preferably by NMR measurements.

The objects of the present invention are also solved by a compound that binds to protein phosphatase 5 in a cleft surrounded by the amino acids Glu427, Val428, Lys429, Pro430, Glu 434, Met454 and Asn456 of the C. *elegans* protein phosphatase 5 amino acid sequence or in a cleft surrounded by the amino acids Glu428, Val429, Lys430, Ala431, Glu 435, Met455 and Asn 457 of the mammalian protein phosphatase 5 amino acid sequence or in a cleft surrounded by the amino acids Glu428, Val429, Lys430, Ala431, Glu 435, Met455 and Asn 457 of the rat protein phosphatase 5 amino acid sequence or in a cleft surrounded by the amino acids Glu428, Val429, Lys430, Ala431, Glu 435, Met455 and Asn 457 of the mouse protein phosphatase 5 amino acid sequence or in a cleft surrounded by the amino acids Glu428, Val429, Lys430, Ala431, Glu 435, Met455 and Asn 457 of the human protein phosphatase 5 amino acid sequence. Preferably, said binding is determined by X-ray crystallography.

The objects of the present invention are also solved by a compound that shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
wherein a result is considered a positive result if the enzymatic activity of protein phosphatase 5 in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested,
wherein said compound binds to protein phosphatase 5 within the region of amino acids 166 to 496 of the *C*. *elegans* protein phosphatase 5 amino acid sequence or within the region of amino acids 166 to 499 of the mammalian protein phosphatase 5 amino acid sequence or within the region of amino acids 166 to 499 of the rat protein phosphatase 5 amino acid sequence or within the region of amino acids 166 to 499 of the mouse protein phosphatase 5 amino acid sequence or within the region of amino acids 166 to 499 of the human protein phosphatase 5 amino acid sequence. Preferably, said binding is determined in an *in vitro* binding assay, more preferably by NMR measurements.

The objects of the present invention are also solved by a compound that shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
wherein a result is considered a positive result if the enzymatic activity of protein phosphatase 5 in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested,
wherein said compound binds to protein phosphatase 5 in a cleft surrounded by the amino acids Glu427, Val428, Lys429, Pro430, Glu 434, Met454 and Asn456 of the C. *elegans* protein phosphatase 5 amino acid sequence or in a cleft surrounded by the amino acids Glu428, Val429, Lys430, Ala431, Glu 435, Met455 and Asn 457 of the mammalian protein phosphatase 5 amino acid sequence or in a cleft surrounded by the amino acids Glu428, Val429, Lys430, Ala431, Glu 435, Met455 and Asn 457 of the rat protein phosphatase 5 amino acid sequence or in a cleft surrounded by the amino acids Glu428, Val429, Lys430, Ala431, Glu 435, Met455 and Asn 457 of the mouse protein phosphatase 5 amino acid sequence or in a cleft surrounded by the amino acids Glu428, Val429, Lys430, Ala431, Glu 435, Met455 and Asn 457 of the human protein phosphatase 5 amino acid sequence. Preferably, said binding is determined by X-ray crystallography.

The objects of the present invention are also solved by a method of screening for compounds that activate protein phosphatase 5, said method comprising
- providing a small molecule compound, preferably a small molecule compound that is not a fatty acid or a derivative thereof,
- determining whether said compound shows a positive result in a phosphatase activation assay with protein phosphatase 5,
   wherein said phosphatase activation assay comprises
   - measuring the enzymatic activity of protein phosphatase 5 in the absence of a compound to be tested, and
   - measuring the enzymatic activity of protein phosphatase 5 under the same conditions, but in the presence of said compound to be tested,
   and wherein a result in said phosphatase activation assay is considered a positive result if the enzymatic activity of protein phosphatase 5 in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested,
- and, preferably, determining in an *in vitro* binding assay, preferably by NMR measurements, if said compound binds to protein phosphatase 5 within the region of amino acids 166 to 496 of the protein phosphatase 5 sequence,
   wherein said compound is considered a successful candidate in said screening method if it shows a positive result in said phosphatase activation assay and, preferably, if it binds to protein phosphatase 5 within the region of amino acids 166 to 496 of the protein phosphatase 5 sequence in said *in vitro* binding assay/in said NMR measurements.

The objects of the present invention are also solved by a method of screening for compounds that activate protein phosphatase 5, said method comprising
- providing a small molecule compound, preferably a small molecule compound that is not a fatty acid or a derivative thereof,
- determining whether said compound shows a positive result in a phosphatase activation assay with protein phosphatase 5,
   wherein said phosphatase activation assay comprises
   - measuring the enzymatic activity of protein phosphatase 5 in the absence of a compound to be tested, and
   - measuring the enzymatic activity of protein phosphatase 5 under the same conditions, but in the presence of said compound to be tested,
   and wherein a result in said phosphatase activation assay is considered a positive result if the enzymatic activity of protein phosphatase 5 in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested,
- and, preferably, determining binding of said compound to protein phosphatase 5 by X ray crystallography,
wherein said compound is considered a successful candidate in said screening method if it shows a positive result in said phosphatase activation assay and, preferably, if it binds to protein phosphatase 5 in a cleft surrounded by the amino acids Glu427, Val428, Lys429, Pro430, Glu 434, Met454 and Asn456 of the *C. elegans* protein phosphatase 5 amino acid sequence or in a cleft surrounded by the amino acids Glu428, Val429, Lys430, Ala431, Glu 435, Met455 and Asn 457 of the mammalian protein phosphatase 5 amino acid sequence or in a cleft surrounded by the amino acids Glu428, Val429, Lys430, Ala431, Glu 435, Met455 and Asn 457 of the rat protein phosphatase 5 amino acid sequence or in a cleft surrounded by the amino acids Glu428, Val429, Lys430, Ala431, Glu 435, Met455 and Asn 457 of the mouse protein phosphatase 5 amino acid sequence or in a cleft surrounded by the amino acids Glu428, Val429, Lys430, Ala431, Glu 435, Met455 and Asn 457 of the human protein phosphatase 5 amino acid sequence.

The following embodiments are, where applicable, embodiments of any of the seven above-described solutions to the objects of the present invention.

Moreover, the following embodiments can, wherever this does not lead to logical contradictions, be combined without restrictions. Thus, the present disclosure embraces, even if not explicitly spelled out in the following, any feasible combination of the following embodiments.

In one embodiment, said protein phosphatase 5 is protein phosphatase 5 from *C. elegans.* In one embodiment, said protein phosphatase 5 is mammalian protein phosphatase 5. In one embodiment, said protein phosphatase 5 is rat protein phosphatase 5. In one embodiment, said protein phosphatase 5 is mouse protein phosphatase 5.In one embodiment, said protein phosphatase 5 is human protein phosphatase 5.

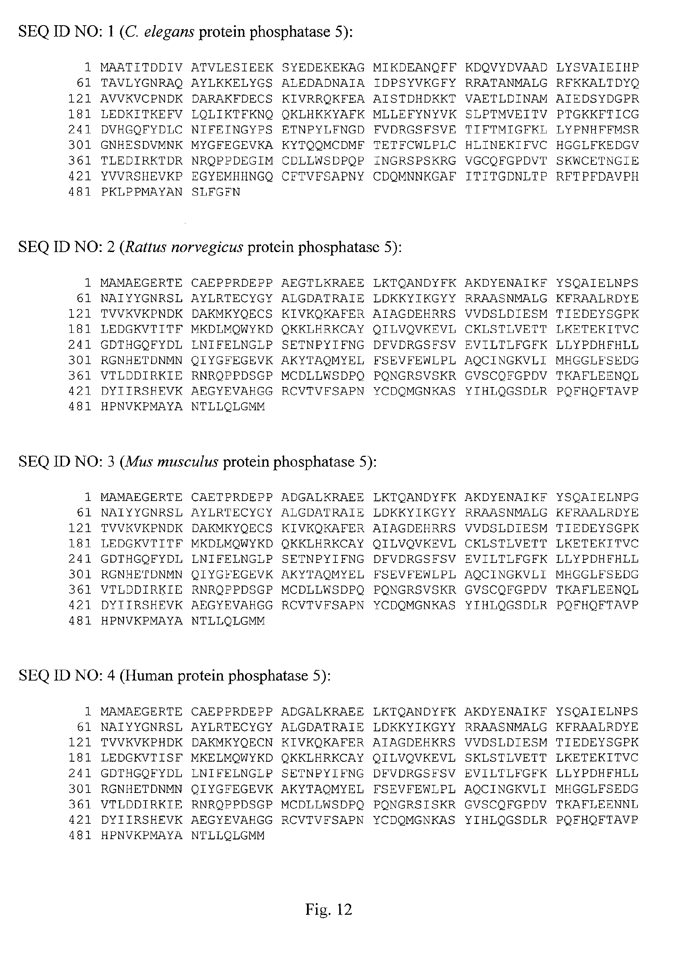

In one embodiment, the amino acid sequence of C. *elegans* protein phosphatase 5 is given by SEQ ID NO: 1. In one embodiment, the amino acid sequence of rat protein phosphatase 5 is given by SEQ ID NO: 2. In one embodiment, the amino acid sequence of mouse protein phosphatase 5 is given by SEQ ID NO: 3. In one embodiment, the amino acid sequence of human protein phosphatase 5 is given by SEQ ID NO: 4. Protein phosphatase 5 sequences of different organisms, corresponding to SEQ ID NO: 1 to 4, are shown in Figure 12.

In one embodiment, said phosphatase activation assay is an *in vitro* assay. Preferably, the enzymatic activity of the phosphatase in the phosphatase activation assay is measured by a colorimetric assay. Preferably, the substrate used in said phosphatase activation assay is para-nitrophenyl phosphate (pNPP). Preferably, said enzymatic activity is measured spectroscopically by the change in absorption at 410 nm. Preferably, the conditions used for measuring the enzymatic activity are 40 mM HEPES/KOH, 20 mM KCl, 5 mM MnCl₂, 1 mM DTT, pH 7.5. Preferably, said phosphatase activation assay is carried out at 20° C. Preferably, the starting concentration of pNPP is 60 mM. Preferably, if the enzymatic activity of protein phosphatase 5 in the presence of the compound to be tested is to be measured, the concentration of the compound to be tested is in the range of 50-500 nM, more preferably in the range of 100-250 nM, more preferably 50, 250 or 500 nM, more preferably 250 nM. In one embodiment, the concentration of the phosphatase is 5-20 µM. In one embodiment, the concentration of the phosphatase is 30 nM.

In one embodiment, in a phosphatase activation assay carried out with protein phosphatase 5 lacking the TPR domain, the enzymatic activity of protein phosphatase 5 lacking the TPR domain in the presence of the compound to be tested is by at least a factor of 1.5, preferably at least a factor of 2, more preferably at least a factor of 3, more preferably at least a factor of 4 lower than in the absence of the compound to be tested. In one embodiment, said protein phosphatase 5 lacking the TPR domain is of the same species as said protein phosphatase 5 used in said phosphatase activation assay with protein phosphatase 5. In one embodiment, said protein phosphatase 5 lacking the TPR domain comprises only amino acids 166 to 499 of the mammalian protein phosphatase 5 sequence. In one embodiment, said protein phosphatase 5 lacking the TPR domain comprises only amino acids 166 to 499 of the rat protein phosphatase 5 sequence. In one embodiment, said protein phosphatase 5 lacking the TPR domain comprises only amino acids 166 to 499 of the mouse protein phosphatase 5 sequence. In one embodiment, said protein phosphatase 5 lacking the TPR domain comprises only amino acids 166 to 499 of the human protein phosphatase 5 sequence. In the above embodiments, said phosphatase activation assay carried out with protein phosphatase 5 lacking the TPR domain preferably comprises the same steps and is carried out under the same conditions as said phosphatase assay with protein phosphatase 5, wherein, preferably, if the enzymatic activity in the presence of the compound to be tested is to be measured, said phosphatase activation assay carried out with protein phosphatase 5 lacking the TPR domain is carried out in the presence of 10 µM, preferably 20 µM, more preferably 50 µM more preferably 100 µM of said compound to be tested.

In one embodiment, said compound does not show a positive result in a phosphatase activation assay with the phosphatase PP1. In one embodiment, said compound does not show a positive result in a phosphatase activation assay with the phosphatase PP2A. In one embodiment, said compound does not show a positive result in a phosphatase activation assay with the phosphatase PP2B/PP3. In one embodiment, said compound does not show a positive result in a phosphatase activation assay with alkaline phosphatase. In one embodiment, said compound shows a positive result with up to 3, preferably up to 2, more preferably up to 1 of the phosphatases included in the group consisting of PP1, PP2A, PP2B/PP3, and alkaline phosphatase. In the above embodiments, said phosphatase assay with PP1, PP2A, PP2B/PP3, or alkaline phosphatase comprises the same steps and is carried out under the same conditions as said phosphatase assay with protein phosphatase 5. Preferably, in said phosphatase activation assay with PP1, PP2A, PP2B/PP3, or alkaline phosphatase a result is considered a positive result if the enzymatic activity of the examined phosphatase in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested.

In one embodiment, said compound does not show a positive result in a phosphatase activation assay with the yeast homolog Ppt1 of protein phosphatase 5. Preferably, in said phosphatase activation assay with Ppt1 a result is considered a positive result if the enzymatic activity of the examined phosphatase in the presence of the compound to be tested is by at least a factor of 1.5, preferably at least a factor of 2, more preferably at least a factor of 3, more preferably at least a factor of 5, more preferably at least a factor of 8, more preferably at least a factor of 10, more preferably at least a factor of 12, more preferably at least a factor of 15, more preferably at least a factor of 20, more preferably at least a factor of 25, more preferably at least a factor of 30, more preferably at least a factor of 40, more preferably at least a factor of 50 higher than in the absence of the compound to be tested.

In one embodiment, said compound binds to protein phosphatase 5 within the region of amino acids 166 to 496 of the protein phosphatase 5 sequence. In one embodiment, said compound binds to protein phosphatase 5 within the region of amino acids 166 to 496 of *C. elegans* protein phosphatase 5. In one embodiment, said compound binds to protein phosphatase 5 within the region of amino acids 166 to 499 of the mammalian protein phosphatase 5 sequence. In one embodiment, said compound binds to protein phosphatase 5 within the region of amino acids 166 to 499 of rat protein phosphatase 5. In one embodiment, said compound binds to protein phosphatase 5 within the region of amino acids 166 to 499 of mouse protein phosphatase 5. In one embodiment, said compound binds to protein phosphatase 5 within the region of amino acids 166 to 499 of human protein phosphatase 5. Preferably, said binding is determined in an *in vitro* binding assay, more preferably by NMR measurements.

In one embodiment, said compound binds to *C. elegans* protein phosphatase 5 within the region of amino acids 166 to 496 of the *C. elegans* protein phosphatase 5 amino acid sequence and to rat protein phosphatase 5 within the region of amino acids 166 to 499 of the rat protein phosphatase 5 amino acid sequence. In one embodiment, said compound binds to *C. elegans* protein phosphatase 5 within the region of amino acids 166 to 496 of the *C. elegans* protein phosphatase 5 amino acid sequence and to mouse protein phosphatase 5 within the region of amino acids 166 to 499 of the mouse protein phosphatase 5 amino acid sequence. In one embodiment, said compound binds to *C. elegans* protein phosphatase 5 within the region of amino acids 166 to 496 of the *C. elegans* protein phosphatase 5 amino acid sequence and to human protein phosphatase 5 within the region of amino acids 166 to 499 of the human protein phosphatase 5 amino acid sequence. In one embodiment, said compound binds to *C. elegans* protein phosphatase 5 within the region of amino acids 166 to 496 of the *C. elegans* protein phosphatase 5 amino acid sequence and to rat, mouse and human protein phosphatase 5 within the region of amino acids 166 to 499 of the rat, mouse or human protein phosphatase 5 amino acid sequence, respectively. Preferably, said binding is determined in an *in vitro* binding assay, more preferably by NMR measurements.

In one embodiment, said compound does not bind to the TPR (tetratricopeptide repeat) domain of protein phosphatase 5. In one embodiment, said compound does not bind to the region of amino acids 1 to 149 of the *C. elegans,* rat, mouse or human protein phosphatase 5 amino acid sequence. Preferably, said failure to bind is determined in an *in vitro* binding assay, more preferably by NMR measurements.

In one embodiment, said compound does not bind to the phosphatase PP1. In one embodiment, said compound does not bind to the phosphatase PP2A. In one embodiment, said compound does not bind to the phosphatase PP2B/PP3. In one embodiment, said compound does not bind to alkaline phosphatase. In one embodiment, said compound binds to up to 3, preferably up to 2, more preferably up to 1 of the phosphatases included in the group consisting of PP1, PP2A, PP2B/PP3, and alkaline phosphatase. In the above embodiments, preferably said failure to bind to PP1, PP2A, or PP2B/PP3 is measured with an *in vitro* binding assay, more preferably by NMR measurements.

In one embodiment, said *in vitro* binding assay is carried out by NMR measurements. Preferably, such NMR measurements are carried out on an 800 MHz spectrometer with a cryoprobe. Preferably, the measurements are carried out in D₂O with phosphate buffer (4 mM KH₂PO₄, 16 mM Na₂HPO₄, 120 mM NaCl, pH 7.4) and, preferably, 1-5% of d6-DMSO. Preferably, measurements are done in high sensitivity experiments with a high number of scans. Preferably, a CPMG sequence with an additional Watergate to suppress residual water is used (this is described in Hajduk et al., 1997; Sklenar et al., 1993). Preferably, peak intensities are normalized by comparing residual DMSO peaks. Preferably, binding is recognized by a reduced peak intensity of certain peaks of the NMR spectrum of the small molecule compound in the presence of the binding protein (e.g. PP5 (166-499)) compared to the NMR spectrum of the small molecule compound in the absence of said binding protein. Preferably, bovine intestinal alkaline phosphatase is used as negative control for the binding protein.

In one embodiment, said compound is a small molecule compound.

In one embodiment, said compound has a molecular weight of up to 2000 Dalton, preferably of up to 1500 Dalton, more preferably of up to 1000 Dalton, more preferably of up to 900 Dalton, more preferably of up to 800 Dalton, more preferably of up to 700 Dalton, more preferably of up to 600 Dalton, more preferably of up to 500 Dalton.

In one embodiment said compound has a molecular weight of at least 100 Dalton, preferably of at least 200 Dalton, more preferably of at least 300 Dalton.

In one embodiment, said compound has a water solubility of at least 50 µg/ml, preferably at least 100 µg/ml, more preferably at least 200 µg/ml, more preferably at least 500 µg/ml, more preferably at least 1 mg/ml, more preferably at least 5 mg/ml, more preferably at least 10 mg/ml, more preferably at least 20 mg/ml, more preferably at least 50 mg/ml, more preferably at least 100 mg/ml, more preferably at least 200 mg/ml, more preferably at least 500 mg/ml, more preferably at least 1000 mg/ml. In one embodiment, said compound has a water solubility of more than 200 µg/ml.

In one embodiment, said method of screening for compounds that activate protein phosphatase 5 further comprises the step of
- determining the solubility of said compound in water,
and said compound is considered a successful candidate only if it has a water solubility of at least 50 µg/ml, preferably at least 100 µg/ml, more preferably at least 200 µg/ml, more preferably at least 500 µg/ml, more preferably at least 1 mg/ml, more preferably at least 5 mg/ml, more preferably at least 10 mg/ml, more preferably at least 20 mg/ml, more preferably at least 50 mg/ml, more preferably at least 100 mg/ml. In a preferred embodiment, said compound is considered a successful candidate only if it has a water solubility of at least 200 µg/ml.

In one embodiment, said compound is a synthetic compound that does not occur in nature. Preferably, said compound is not a derivative of a compound occurring in nature.

In one embodiment, said compound is not a fatty acid. In one embodiment, said compound is not a fatty acid or a derivative thereof. In one embodiment, said compound is not a lipid containing a fatty acid.

In one embodiment, said compound is not a polyunsaturated fatty acid. In one embodiment, said compound is not a polyunsaturated fatty acid or a derivative thereof. In one embodiment, said compound is not a lipid containing a polyunsaturated fatty acid.

In one embodiment, said compound is not arachidonic acid or a derivative thereof. In one embodiment, said compound is not arachidonic acid, linoleic acid, oleic acid, linolenic acid, or a derivative thereof.

In one embodiment, said compound is not a peptide or a derivative thereof. In one embodiment, said compound does not comprise amino acids.

In one embodiment, said binding of said compound to protein phosphatase 5 does not affect binding of Hsp90 to protein phosphatase 5, as determined by sedimentation velocity ultracentrifugation.

In one embodiment, the enzymatic activity of said protein phosphatase 5 in the presence of said compound, as determined by said phosphatase assay, is not significantly affected by the presence or absence of a peptide with the sequence MMEVD at a concentration of 60 µM.

In one embodiment, said compound does not affect the ATPase activity of the human chaperone protein HSP-1, of the *C. elegans* chaperone protein Hsc70, of the *C. elegans* chaperone protein Hsc70 in the presence of the cochaperones DNJ-13 and BAG-1, of the human chaperone protein Hsp90, of the *C. elegans* chaperone protein DAF-21, and/or of the *C. elegans* chaperone protein DAF-21 in the presence of the cochaperone AHA-1 in an *in vitro* ATPase assay.

In one embodiment, said ATPase activity is measured with an ATPase assay that is carried out as a regenerative assay, wherein ADP produced during the ATPase reaction is recycled to ATP with the help of lactate dehydrogenase, NADH, phosphoenolpyruvate and pyruvate kinase. This changes the absorbance at 340 nm, when NADH is oxidized to NAD⁺, giving a sensitive signal for the determination of activity. Preferably, the change in absorbance at 340 nm that occurs during oxidizing of NADH to NAD⁺ is measured by spectroscopic measures. Preferably, said *in vitro* ATPase assay is carried out at 30 °C. Preferably, said *in vitro* ATPase assay is carried out in the presence of 3 µM or the chaperone protein. Preferably the concentration of each of the cochaperones present is 5 µM. Preferably, the chaperones and cochaperones used are bacterially expressed proteins.

In one embodiment, said compound has the general structure
*A-B* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl, preferably an aldehyde, ketone, ester or amide,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl, wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
      wherein, preferably, one of the substituents is Cl in para-position to *B,*
      wherein, preferably, A is phenyl substituted with one or more substituents, preferably with one substituent,
      wherein, preferably, at least one substituent is in para-position to *B,*
      wherein, preferably, the one or more substituents are independently selected from the group consisting of F, Cl, and Br, wherein, more preferably, the one or more substituents are all Cl,
      wherein, most preferably, A is 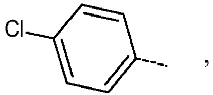
and wherein *B* is 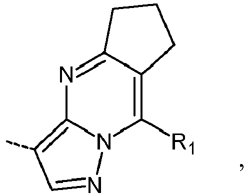 wherein R₁ is 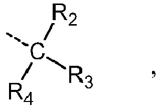 wherein R₂, R₃ and R₄ are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
wherein, preferably, each of R₂, R₃ and R₄ comprises up to 14, preferably up to 10, more preferably up to 6, more preferably up to 3, more preferably up to 2, more preferably up to 1 carbon atoms,
wherein, preferably, *B* is 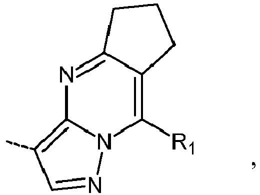 wherein R₁ is 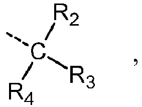 wherein R₂, R₃ and R₄ are independently selected from the group consisting of F, Cl, and Br, wherein, preferably, at least one of R₂, R₃ and R₄, more preferably at least two of R₂, R₃ and R₄ are F,
wherein, more preferably, *B* is

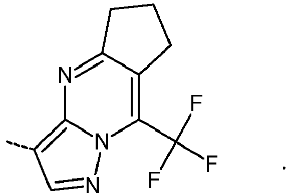

In one preferred embodiment, said compound has the structure represented by Formula I:

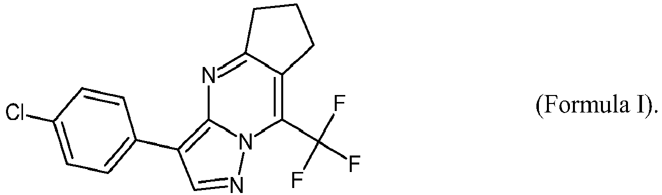

In one embodiment, said compound has the general structure
*A-B-C-D* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is Cl in para-position to *B,*
wherein, preferably, A is phenyl substituted with one or more substituents, preferably with one substituent,
wherein, preferably, at least one substituent is in para-position to *B,* wherein, preferably, the one or more substituents are independently selected from the group consisting of F, Cl, and Br, wherein, more preferably, the one or more substituents are all Cl,
wherein, most preferably, A is 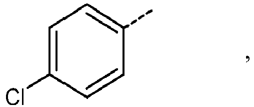 wherein *B* is 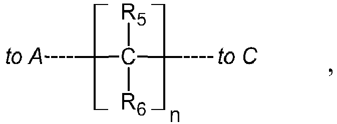 wherein n is from 1 to 5, preferably from 1 to 4, more preferably from 1 to 3, more preferably 1 or 2, more preferably 1,
and R₅ and R₆ are selected, independently for each occurrence of R₅ resp. R₆, from the group consisting of hydrogen and methyl,
wherein, preferably, *B* is 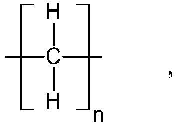 wherein n is from 1 to 3, preferably 1 or 2, more preferably 1,
wherein, most preferably, *B* is 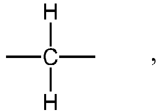 wherein C is 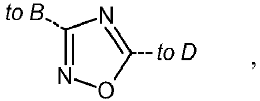 and wherein D is
- unsubstituted phenyl or
- phenyl substituted with one or more substituents,
   wherein the one or more substituents are independently selected from the group consisting of
- halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one substituent is a methoxy group in meso-position to Cand one substituent is a methoxy group in para-position to C,
wherein, preferably, *D* is phenyl substituted with one or more substituents, more preferably substituted with two substituents,
wherein, preferably, one substituent is in meso-position to *C* and one in para-position to *C*,
wherein, preferably, the one or more substituents are independently selected from the group consisting of straight alkoxy groups,
wherein, preferably, each of the one or more substituents comprises up to 3, preferably up to 2, more preferably up to 1 carbon atoms,
wherein, most preferably, D is

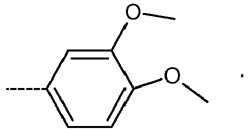

In one preferred embodiment, said compound has the structure represented by Formula II:

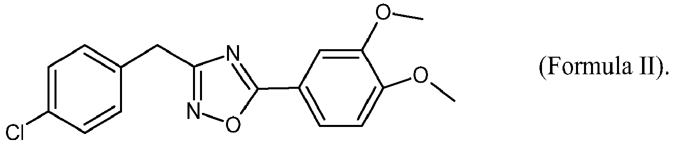

In one embodiment, said compound has the general structure
*A-B-C-D* ,
wherein A comprises a non-planar group comprising 6 to 13 carbon atoms, preferably 8 to 12 carbon atoms, more preferably 9-11 carbon atoms, more preferably 10 carbon atoms and has, preferably, a molecular weight of up to 300 Dalton,
wherein, preferably, A is a triyclic group,
wherein, preferably, A is composed exclusively of carbon and hydrogen atoms,
wherein, most preferably, A is 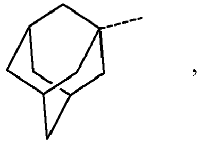 wherein *B* is 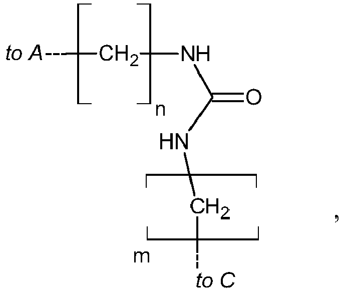 wherein n is from 0 to 4, preferably 0 or 1,
and wherein m is from 0 to 4, preferably from 0 to 2, more preferably 1 or 2, more preferably 1,
wherein, preferably, *B* is 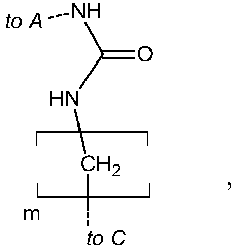 wherein m is from 0 to 4, preferably from 0 to 2, more preferably 1 or 2,
wherein, most preferably, *B* is 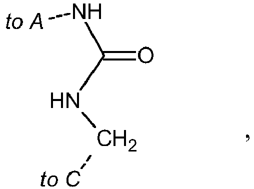 wherein C is 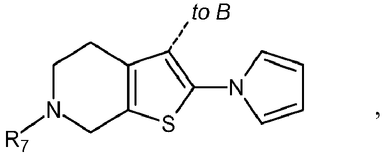 wherein R₇ is a straight or branched alkyl chain with up to 4 carbon atoms, preferably an alkyl chain with up to 2 carbon atoms, most preferably a methyl group.

In one preferred embodiment, said compound has the structure represented by Formula III:

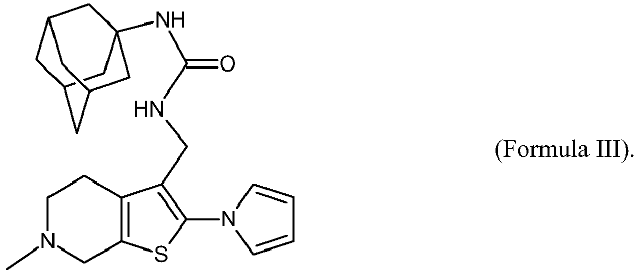

In one embodiment, said compound has the general structure
*A-B-C-D* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is F in para-position to *B,*
wherein, preferably, A is phenyl substituted with one or more substituents, preferably with one substituent,
wherein, preferably, at least one substituent is in para-position to *B,* wherein, preferably, the one or more substituents are independently selected from the group consisting of F, Cl, and Br, wherein, more preferably, the one or more substituents are all F,
wherein, most preferably, A is 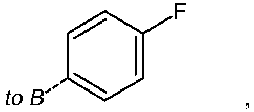 wherein *B* is 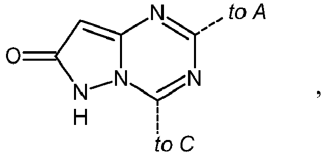 wherein C is 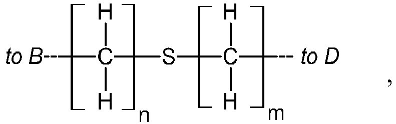 wherein n is from 0 to 4, preferably 0 or 1,
and wherein m is from 0 to 4, preferably from 0 to 2,
wherein, preferably, C is 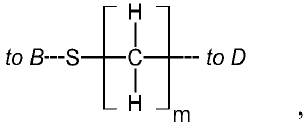 wherein m is from 0 to 4, preferably from 0 to 2, more preferably 1 or 2,
wherein, most preferably, C is 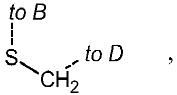 and wherein D is
- unsubstituted phenyl or
- phenyl substituted with one or more substituents, wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
   wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
   wherein, preferably, one of the substituents is Br in para-position to *C,*
   wherein, preferably, *D* is phenyl substituted with one or more substituents, preferably with one substituent,
   wherein, preferably, at least one substituent is in para-position to *C,* wherein, preferably, the one or more substituents are independently selected from the group consisting of F, Cl, and Br, wherein, more preferably, the one or more substituents are all Br,
   wherein, most preferably, *D* is

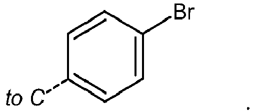

In one preferred embodiment, said compound has the structure represented by Formula IV:

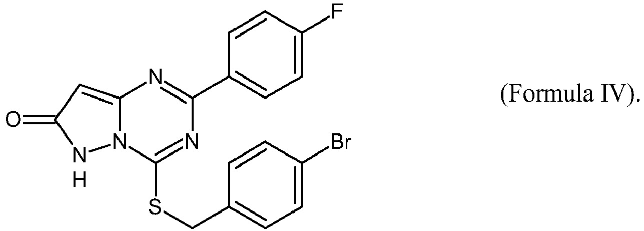

In one embodiment, said compound has the general structure
*A-B-C-D-C-B-A*
or
*A-B-C-D-*R₉ ,
wherein A is 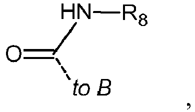 wherein R₈ is a straight or branched alkyl chain with up to 4 carbon atoms, preferably an alkyl chain with up to 2 carbon atoms, most preferably a methyl group,
wherein *B* is 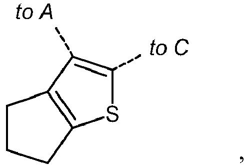 wherein C is 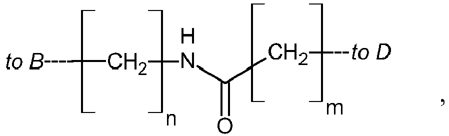 wherein n is from 0 to 4, preferably 0 or 1,
and wherein m is from 0 to 4, preferably from 0 to 2,
wherein, preferably, C is 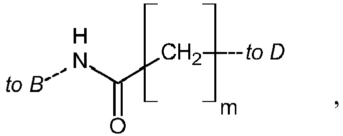 wherein m is from 0 to 4, preferably from 0 to 2, more preferably 1 or 2,
wherein, most preferably, C is 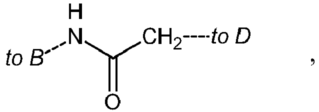 and wherein D is 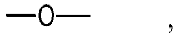 wherein A is the same or different for each occurrence,
wherein B is the same or different for each occurrence,
wherein C is the same or different for each occurrence,
wherein R₉ is hydrogen, methyl or straight or branched alkyl which is unsubstituted or substituted with one or more substituents selected from the group consisting of halogen, preferably F, Cl, Br, or I, hydroxyl, amino, nitro, cyano, thiol, sulfonyl, carbonyl, carboxyl, substituted or unsubstituted aryl, preferably substituted or unsubstituted phenyl, and substituted or unsubstituted heteroaryl,
wherein, preferably, the molecular weight of R₉ is below 2000 Dalton, more preferably below 1000 Dalton, more preferably below 500 Dalton, more preferably below 450 Dalton, more preferably below 250 Dalton, more preferably below 200 Dalton, more preferably below 150 Dalton, more preferably below 100 Dalton, more preferably below 50 Dalton.

In one preferred embodiment, said compound has the structure represented by Formula V:

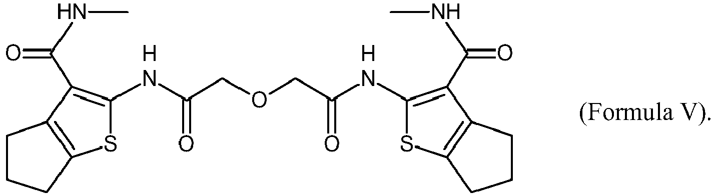

In one embodiment, any of the above-mentioned compounds is for use in the treatment of a tauopathy,
preferably in the treatment of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia or white matter tauopathy with globular glial inclusions.

In one embodiment, any of the above-mentioned compounds is for use in the treatment of a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions.

In one embodiment, any of the above-mentioned compounds is for use in the treatment of Alzheimer's disease. In one embodiment, any of the above-mentioned compounds is for use in the treatment of chronic traumatic encephalopathy. In one embodiment, any of the above-mentioned compounds is for use in the treatment of frontotemporal dementia. In one embodiment, any of the above-mentioned compounds is for use in the treatment of parkinsonism linked to chromosome 17 (caused by MAPT mutations). In one embodiment, any of the above-mentioned compounds is for use in the treatment of frontotemporal lobar degeneration, preferably caused by C9ORF72 mutations. In one embodiment, any of the above-mentioned compounds is for use in the treatment of Niemann-Pick disease type C. In one embodiment, any of the above-mentioned compounds is for use in the treatment of Pick's disease. In one embodiment, any of the above-mentioned compounds is for use in the treatment of progressive supranuclear palsy. In one embodiment, any of the above-mentioned compounds is for use in the treatment of tangle-only dementia. In one embodiment, any of the above-mentioned compounds is for use in the treatment of white matter tauopathy with globular glial inclusions.

In one embodiment, any of the above-mentioned compounds is for use in the treatment of cancer,
preferably in the treatment of a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina.

In one embodiment, any of the above-mentioned compounds is for use in the treatment of a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor.

In one embodiment, any of the above-mentioned compounds is for use in the treatment of pancreatic carcinoma.

In one embodiment, said compound is administered to a patient. Preferably, an effective amount of said compound is administered to a patient. Preferably, said patient is a mammal. More preferably, said patient is a rat, a mouse or a human. More preferably, said patient is a human.

In one embodiment, said administration occurs by oral administration, by parenteral administration, by intravenous injection, by intradermal injection, or by topical administration. In one embodiment, said administration occurs by a series of injections or continuous infusion over an extended period of time.

The objects of the present invention are also solved by a method for activating protein phosphatase 5, said method comprising the steps of contacting protein phosphatase 5 with a compound as defined above in an amount sufficient to activate protein phosphatase 5.

In such method, said compound and said protein phosphatase 5 are as defined in the embodiments above.

The objects of the present invention are also solved by a pharmaceutical composition comprising one or more compounds as defined above.

In such pharmaceutical composition, said compound(s) and said protein phosphatase 5 are as defined in the embodiments above.

The objects of the present invention are also solved by a method of using a compound as defined above for treating a patient who is suffering from a tauopathy,
preferably a patient who is suffering from a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a patient who is suffering from a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or for treating a patient who is suffering from cancer,
preferably a patient who is suffering from a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a patient who is suffering from a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably a patient who is suffering from pancreatic carcinoma.

In such method, said compound and said patient are as defined in the embodiments above.

The objects of the present invention are also solved by a method for treatment of a patient who is suffering from a tauopathy,
preferably a patient who is suffering from a disease selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a patient who is suffering from a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or for treatment of a patient who is suffering from cancer,
preferably a patient who is suffering from a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a patient who is suffering from a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably a patient who is suffering from pancreatic carcinoma,
said method comprising the steps of:
- obtaining a compound as defined in the embodiments above,
- administering said compound to said patient.

In such method, said compound, said patient, and said administering are as defined in the embodiments above.

Preferably, an effective amount of said compound is administered to said patient.

The objects of the present invention are also solved by the use of a compound as defined in the embodiments above for the manufacture of a medicament or a pharmaceutical composition for treating a patient who is suffering from a tauopathy, preferably from a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a patient who is suffering from a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or for treating a patient who is suffering from cancer,
preferably a patient who is suffering from a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a patient who is suffering from a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably a patient who is suffering from pancreatic carcinoma.

In such method, said compound and said patient are as defined in the embodiments above.

The objects of the present invention are also solved by a compound with the general structure
*A-B* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl, preferably an aldehyde, ketone, ester or amide,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
      wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
      wherein, preferably, one of the substituents is Cl in para-position to *B,*
      wherein, preferably, A is phenyl substituted with one or more substituents, preferably with one substituent,
      wherein, preferably, at least one substituent is in para-position to *B,* wherein, preferably, the one or more substituents are independently selected from the group consisting of F, Cl, and Br, wherein, more preferably, the one or more substituents are all Cl,
      wherein, most preferably, A is 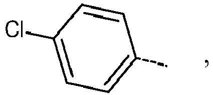 and wherein *B* is 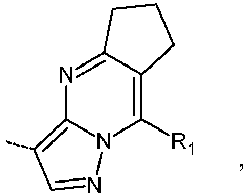 wherein R₁ is 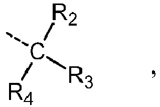
   wherein R₂, R₃ and R₄ are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   wherein, preferably, each of R₂, R₃ and R₄ comprises up to 14, preferably up to 10, more preferably up to 6, more preferably up to 3, more preferably up to 2, more preferably up to 1 carbon atoms,
   wherein, preferably, *B* is

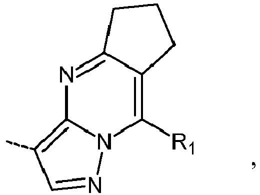

   wherein R₁ is 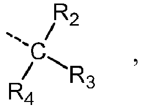 wherein R₂, R₃ and R₄ are independently selected from the group consisting of F, Cl, and Br, wherein, preferably, at least one of R₂, R₃ and R₄, more preferably at least two of R₂, R₃ and R₄ are F,
   wherein, more preferably, *B* is 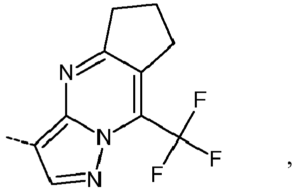 wherein, most preferably, said compound has the structure represented by Formula I:

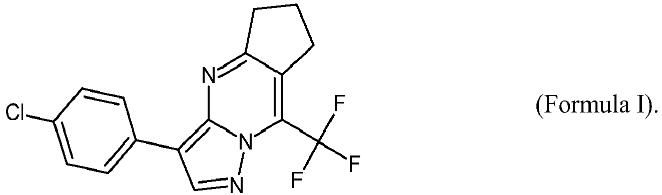

Preferably, said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

The objects of the present invention are also solved by the use of a compound as defined above in a method for increasing the activity of protein phosphatase 5.

The objects of the present invention are also solved by a compound with the general structure
*A-B-C-D* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
   wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is Cl in para-position to *B,*
wherein, preferably, A is phenyl substituted with one or more substituents, preferably with one substituent,
wherein, preferably, at least one substituent is in para-position to *B,* wherein, preferably, the one or more substituents are independently selected from the group consisting of F, Cl, and Br, wherein, more preferably, the one or more substituents are all Cl,
wherein, most preferably, A is 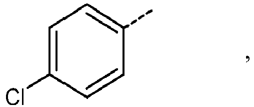 wherein *B* is 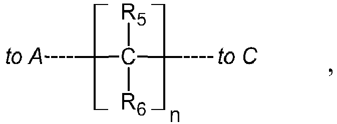 wherein n is from 1 to 5, preferably from 1 to 4, more preferably from 1 to 3, more preferably 1 or 2, more preferably 1,
and R₅ and R₆ are selected, independently for each occurrence of R₅ resp. R₆, from the group consisting of hydrogen and methyl,
wherein, preferably, *B* is 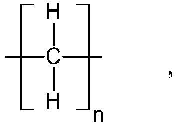 wherein n is from 1 to 3, preferably 1 or 2, more preferably 1,
wherein, most preferably, *B* is 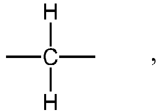 wherein C is 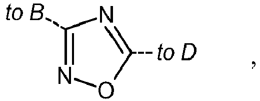 and wherein D is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one substituent is a methoxy group in meso-position to Cand one substituent is a methoxy group in para-position to C,
wherein, preferably, D is phenyl substituted with one or more substituents, more preferably substituted with two substituents,
wherein, preferably, one substituent is in meso-position to Cand one in para-position to C,
wherein, preferably, the one or more substituents are independently selected from the group consisting of straight alkoxy groups,
wherein, preferably, each of the one or more substituents comprises up to 3, preferably up to 2, more preferably up to 1 carbon atoms,
wherein, most preferably, D is 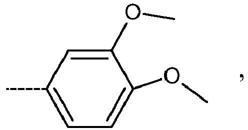 wherein, most preferably, said compound has the structure represented by Formula II:

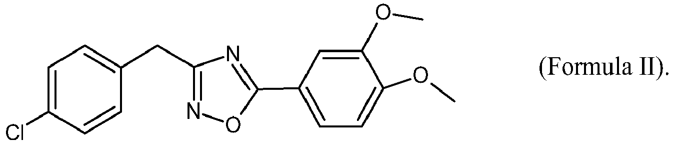

Preferably, said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

The objects of the present invention are also solved by the use of a compound as defined above in a method for increasing the activity of protein phosphatase 5.

The objects of the present invention are also solved by a compound with the general structure
*A-B-C-D* ,
wherein A comprises a non-planar group comprising 6 to 13 carbon atoms, preferably 8 to 12 carbon atoms, more preferably 9-11 carbon atoms, more preferably 10 carbon atoms and has, preferably, a molecular weight of up to 300 Dalton,
wherein, preferably, A is a triyclic group,
wherein, preferably, A is composed exclusively of carbon and hydrogen atoms,
wherein, most preferably, A is 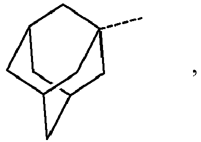 wherein *B* is 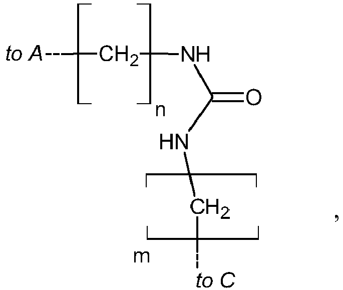 wherein n is from 0 to 4, preferably 0 or 1,
and wherein m is from 0 to 4, preferably from 0 to 2, more preferably 1 or 2, more preferably 1,
wherein, preferably, *B* is 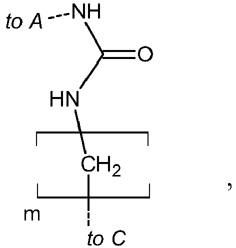 wherein m is from 0 to 4, preferably from 0 to 2, more preferably 1 or 2,
wherein, most preferably, *B* is 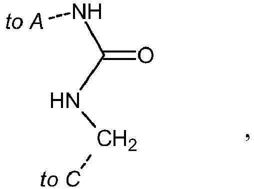 wherein C is 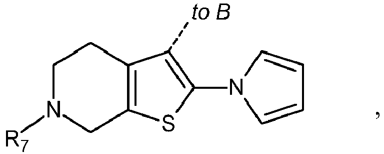 wherein R₇ is a straight or branched alkyl chain with up to 4 carbon atoms, preferably an alkyl chain with up to 2 carbon atoms, most preferably a methyl group,
wherein, most preferably, said compound has the structure represented by Formula III:

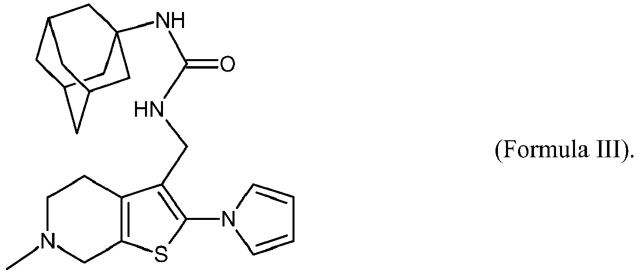

Preferably, said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

The objects of the present invention are also solved by the use of a compound as defined above in a method for increasing the activity of protein phosphatase 5.

The objects of the present invention are also solved by a compound with the general structure
*A-B-C-D* ,
wherein A is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is F in para-position to *B,*
wherein, preferably, A is phenyl substituted with one or more substituents, preferably with one substituent,
wherein, preferably, at least one substituent is in para-position to *B,*
wherein, preferably, the one or more substituents are independently selected from the group consisting of F, Cl, and Br, wherein, more preferably, the one or more substituents are all F,
wherein, most preferably, A is 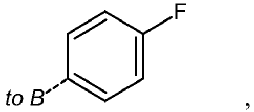 wherein *B* is 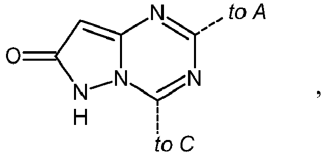 wherein C is 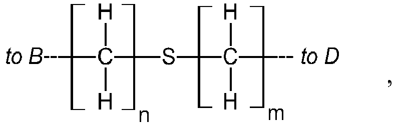 wherein n is from 0 to 4, preferably 0 or 1,
and wherein m is from 0 to 4, preferably from 0 to 2,
wherein, preferably, C is 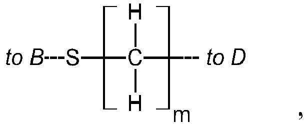 wherein m is from 0 to 4, preferably from 0 to 2, more preferably 1 or 2,
wherein, most preferably, C is 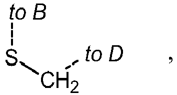 and wherein D is
- unsubstituted phenyl or
- phenyl substituted with one or more substituents,
   wherein the one or more substituents are independently selected from the group consisting of
   - halogen (preferably F, Cl, Br, or I),
   - hydroxyl,
   - amino,
   - nitro,
   - cyano,
   - thiol,
   - sulfonyl,
   - carbonyl,
   - carboxyl,
   - straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
   - substituted or unsubstituted cycloalkyl, and
   - substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms, wherein, preferably, one of the substituents is Br in para-position to C,
wherein, preferably, D is phenyl substituted with one or more substituents, preferably with one substituent,
wherein, preferably, at least one substituent is in para-position to C, wherein, preferably, the one or more substituents are independently selected from the group consisting of F, Cl, and Br, wherein, more preferably, the one or more substituents are all Br,
wherein, most preferably, D is 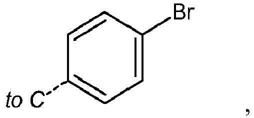 wherein, most preferably, said compound has the structure represented by Formula IV:

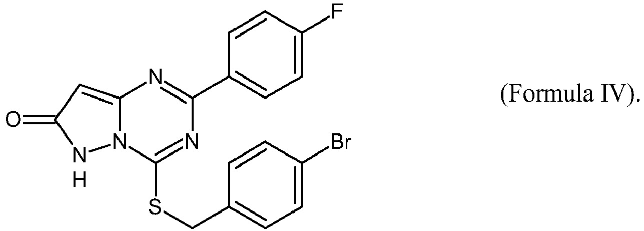

Preferably, said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

The objects of the present invention are also solved by the use of a compound as defined above in a method for increasing the activity of protein phosphatase 5.

The objects of the present invention are also solved by a compound with the general structure
*A-B-C-D-C-B-A*
or
*A-B-C-D-R*₉ ,
wherein A is 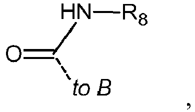 wherein R₈ is a straight or branched alkyl chain with up to 4 carbon atoms, preferably an alkyl chain with up to 2 carbon atoms, most preferably a methyl group,
wherein *B* is 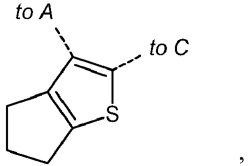 wherein C is 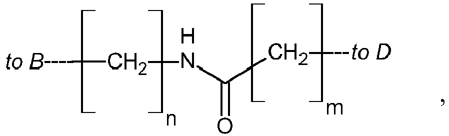 wherein n is from 0 to 4, preferably 0 or 1,
and wherein m is from 0 to 4, preferably from 0 to 2,
wherein, preferably, C is 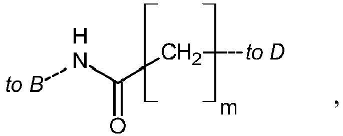 wherein m is from 0 to 4, preferably from 0 to 2, more preferably 1 or 2,
wherein, most preferably, C is 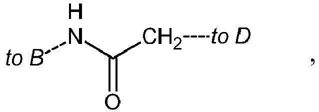 and wherein D is 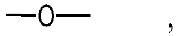 wherein A is the same or different for each occurrence,
wherein B is the same or different for each occurrence,
wherein C is the same or different for each occurrence,
wherein R₉ is hydrogen, methyl or straight or branched alkyl which is unsubstituted or substituted with one or more substituents selected from the group consisting of halogen, preferably F, Cl, Br, or I, hydroxyl, amino, nitro, cyano, thiol, sulfonyl, carbonyl, carboxyl, substituted or unsubstituted aryl, preferably substituted or unsubstituted phenyl, and substituted or unsubstituted heteroaryl,
wherein, preferably, the molecular weight of R₉ is below 2000 Dalton, more preferably below 1000 Dalton, more preferably below 500 Dalton, more preferably below 450 Dalton, more preferably below 250 Dalton, more preferably below 200 Dalton, more preferably below 150 Dalton, more preferably below 100 Dalton, more preferably below 50 Dalton,
wherein, most preferably, said compound has the structure represented by Formula V:

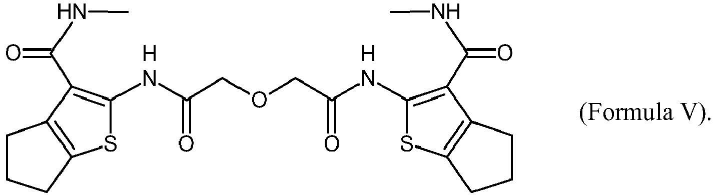

Preferably, said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

The objects of the present invention are also solved by the use of a compound as defined above in a method for increasing the activity of protein phosphatase 5.

The term "protein phosphatase 5", as used herein, refers to an enzyme, preferably an enzyme with E.C. number 3.1.3.16, that catalyzes the removal of phosphate groups from certain substrates (i.e. the dephosphorylation of these substrates) by hydrolyzing phosphoric acid monoesters into a phosphate ion and a molecule with a free hydroxyl group. The polypeptide sequence of protein phosphatase 5 includes a regulatory "TPR domain" ("tetratricopeptide repeat domain") which comprises amino acids 1 to 150 in the C. *elegans* protein phosphatase 5 sequence given by SEQ ID NO: 1, amino acids 1 to 150 in the rat protein phosphatase 5 sequence given by SEQ ID NO: 2, amino acids 1 to 150 in the mouse protein phosphatase 5 sequence given by SEQ ID NO: 3, or amino acids 1 to 150 in the human protein phosphatase 5 sequence given by SEQ ID NO: 4. The polypeptide sequence of protein phosphatase 5 further includes a "phosphatase domain" which is responsible for the enzymatic activity of protein phosphatase 5 and comprises amino acids 166 to 490 in the C. *elegans* protein phosphatase 5 sequence given by SEQ ID NO: 1, amino acids 166 to 491 in the rat protein phosphatase 5 sequence given by SEQ ID NO: 2, amino acids 166 to 491 in the mouse protein phosphatase 5 sequence given by SEQ ID NO: 3, and amino acids 166 to 491 in the human protein phosphatase 5 sequence given by SEQ ID NO: 4.

The terms "PPP", "PP2A", PP2B/PP3, "alkaline phosphatase", "Hsc70" and "Hsp90" are known to the person of skill in the art and designate protein phoshatase 1 (PP1), protein phoshatase 2A (PP2A), protein phoshatase 2B/protein phoshatase 3 (PP2B/PP3), alkaline phosphatase, heat stress cognate 70 (Hsc70), and heat shock protein 90 (Hsp90), respectively.

As used herein, the term "small molecule compound" refers to a compound with a molecular weight of up to 900 Dalton.

The term "derivate", as used for example in the context of a derivate of a fatty acid, a polyunsaturated fatty acid, arachidonic acid, linoleic acid, oleic acid, linolenic acid or a peptide, refers to derivates such as esters of such fatty acid, polyunsaturated fatty acid, arachidonic acid, linoleic acid, oleic acid, linolenic acid or peptide, respectively. In one embodiment, the term "derivate" of a fatty acid, a polyunsaturated fatty acid, arachidonic acid, linoleic acid, oleic acid, linolenic acid or a peptide, refers to an ester, preferably an ethyl ester or an ester with Coenzyme A, of said fatty acid, polyunsaturated fatty acid, arachidonic acid, linoleic acid, oleic acid, linolenic acid or peptide.

The term "phosphatase activation assay", as used herein, refers to a biochemical assay that is adapted to determine whether changes in a certain parameter of a phosphatase reaction result in changes in the enzymatic activity of a certain phosphatase. For example, a phosphatase activation assay may allow to determine whether the presence of a certain compound results in changes in the enzymatic activity of a phosphatase, as compared to in the absence of that compound.

"Enzymatic activity", as used herein, refers to the efficiency with which an enzyme catalyzes the chemical reaction that it is adapted to catalyze. If used in connection with protein phosphatase 5, the term refers to the efficiency with which protein phosphatase 5 catalyzes the removal of phosphate groups from a substrate (i.e. dephosphorylation of that substrate) under certain conditions.

A compound is said to "activate" an enzyme, if the enzyme shows an increased enzymatic activity in the presence of that compound, as compared to in the absence of that compound. For example, a compound is said to "activate" protein phosphatase 5, if the efficiency with which protein phosphatase 5 catalyzes dephosphorylation of its substrates is increased in the presence of said compound, as compared to the same conditions, but in the absence of said compound.

At some instances, the present application refers to "measuring the enzymatic activity" of an enzyme. As used herein, this means that a measured value that is proportional to or reflects the enzymatic activity of that enzyme is measured. For example, the present application may refer to "measuring the enzymatic activity of protein phosphatase 5". Typically, this means that an *in vitro* reaction of protein phosphatase 5 with a suitable substrate is carried out.As the substrate para-nitrophenyl phosphate (pNPP) is used. The conditions for the phosphatase reaction are 40 mM HEPES/KOH, 20 mM KCl, 5 mM MnCl₂, 1 mM DTT, pH 7.5. The reaction is carried out at 20° C. The starting concentration of pNPP is 60 mM. The concentration of the compound to be tested, if present, is in the range of 50-500 nM, preferably in the range of 100-250 nM, more preferably 50, 250 or 500 nM, more preferably 250 nM. As phosphatase, bacterially expressed and purified phosphatase is used. The concentration of the phosphatase in the reaction is 30 nM. The rate at which the dephosphorylation of this substrate proceeds is monitored, typically by measuring the rate of formation of the reaction product by spectroscopic measurements (dephosphorylation of pNPP, e.g., results in generation of p-nitrophenol, a chromogenic product with strong absorbance at about 410 nm (extinction coefficient ε = 15100 cm⁻¹M⁻¹), thus allowing to monitor generation of the reaction product by monitoring the absorbance at 410 nm). If the reaction is carried out in the presence of an excess of substrate, the initial velocity at which the reaction product is generated (i.e. the slope of the initial phase of the curve in a graph plotting the absorbance at 410 nm over time) is proportional to the enzymatic activity of protein phosphatase 5under the specific reaction conditions used. Alternatively, after starting the phosphatase reaction the reaction may be allowed to proceed for a defined incubation period (usually 30 minutes), then the reaction is stopped (for example by addition of a small volume of 1 M NaOH), and the absorbance at 410 nm is measured. Upon subtraction of the absorbance of a negative control (a reaction carried out with a non-functional, for example pre-boiled, phosphatase), the obtained value is proportional to the enzymatic activity of protein phosphatase 5 under the specific reaction conditions used (since the reaction is carried out in the presence of an excess of substrate and the incubation period is chosen such that the reaction is stopped while it is still in the quasi-linear Phase).

At some instances, the present application refers to the enzymatic activity of an enzyme being under certain conditions "higher" by a certain factor than under certain other conditions. For example, the present application may state that the enzymatic activity of protein phosphatase 5 in the presence of a certain compound is higher by at least a factor of 10 than in the absence of that compound. As used herein, this means that, if the enzymatic activity of protein phosphatase 5 is measured (a) under certain conditions in the presence of that compound and (b) under the same conditions, but in the absence of that compound, and if the value obtained under (a) for the enzymatic activity of protein phosphatase 5 in the presence of that compound is divided by the value obtained under (b) for the enzymatic activity of protein phosphatase 5 in the absence of that compound, this results in a value of 10 or higher. On the other hand, the present application may state that the enzymatic activity of protein phosphatase 5 in the presence of a certain compound is lower by at least a factor of 2 than in the absence of that compound. As used herein, this means that, if the enzymatic activity of protein phosphatase 5 is measured (a) under certain conditions in the presence of that compound and (b) under the same conditions, but in the absence of that compound, and if the value obtained under (b) for the enzymatic activity of protein phosphatase 5 in the absence of that compound is divided by the value obtained under (a) for the enzymatic activity of protein phosphatase 5 in the presence of that compound, this results in a value of 2 or higher.

"Sedimentation velocity ultracentrifugation" is considered as a method to determine whether binding of a compound to protein phosphatase 5 affects binding of another protein (such as Hsp90) to protein phosphatase 5 only, if it is explicitly referred to. According to the application, such sedimentation velocity ultracentrifugation is typically carried out as described in the section "Analytical ultracentrifugation (AUC)" of Example 1.

If the present application states that a compound "has a water solubility of at least *n* mg/ml", this means that a compound dissolves in water at an amount of at least *n* mg/ml at a temperature of 25 °C. If the present application states that a compound "has a water solubility of more than *n* mg/ml", this means that a compound dissolves in water at an amount of more than *n* mg/ml at a temperature of 25 °C.

Solubility measurements can be performed by the shake-flask method under equilibrium conditions at 25 °C (pH 7.4). A small amount of powder of the test compound, preferably 10 µg or 1 mg of powder of the test compound, is dissolved in water (excess of solid). This solution is shaken at 25 °C for 24 h or until equilibrium is reached. After separation of the resulting slurry by filtration (e.g. 0.2 µm), the absorption of the filtrate is measured by UV/Vis spectrometry, and the concentration of the compound in the filtrate (which equals the water solubility of the compound) is calculated using a previously determined specific absorption coefficient.

As used herein, the term *"in vitro"* means occurring outside of a living organism. In some embodiments, the term refers to processes or conditions that are both outside of a living organism and not in a cell culture system.

ATPase activity can be measured as described in Gaiser et al., 2009 with an ATPase assay that is carried out as a regenerative assay, wherein ADP produced during the ATPase reaction is recycled to ATP with the help of lactate dehydrogenase, NADH, phosphoenolpyruvate and pyruvate kinase. This changes the absorbance at 340 nm, when NADH is oxidized to NAD⁺, giving a sensitive signal for the determination of activity. The change in absorbance at 340 nm that occurs during oxidizing of NADH to NAD⁺ is measured by spectroscopic measures. Preferably, said *in vitro* ATPase assay is carried out at 30 °C. Preferably, said *in vitro* ATPase assay is carried out in the presence of 3 µM or the chaperone protein. Preferably the concentration of each of the cochaperones present is 5 µM. Preferably, the chaperones and cochaperones used are bacterially expressed proteins.

At some instances, the present application refers to a binding (or failure to bind) being determined/measured "by NMR measurements". Such NMR measurements can for example be carried out on an 800 MHz spectrometer (Bruker) with cryoprobe. The measurements can be done in D₂O with phosphate buffer (4 mM KH₂PO₄, 16 mM Na₂HPO₄, 120 mM NaCl, pH 7.4) and 1-5% of d6-DMSO. Preferably, measurements are done in high sensitivity experiments with a high number of scans. Preferably, a CPMG sequence with an additional Watergate to suppress residual water is used (Hajduk et al., 1997; Sklenar et al., 1993). Peak intensities can be normalized by comparing residual DMSO peaks. Preferably, binding is recognized by a reduced peak intensity of certain peaks of the NMR spektrum of the small molecule compound in the presence of the binding protein (e.g. PP5 (166-499)) compared to the NMR spektrum of the small molecule compound in the absence of said binding protein. (This is caused by a much slower tumbling rate of the protein-ligand complex in comparison to the free small molecule compound. Some peaks of the small molecule compound may remain unreduced, indicating that certain moieties of the P5SA-2 molecule are still able to rotate freely.) Preferably, bovine intestinal alkaline phosphatase is used as negative control for the binding protein.

At some instances, the present application refers to a binding being "determined by X-ray crystallography". Such X-ray crystallography can be carried out as described in Example 1 in the section "Crystallization and structure determination".

"Tau", as used herein, designates the microtubule-associated protein tau that is found in neurons and stimulates and maintains the assembly and stability of microtubules (MG Spillanti, M Goedert (2013) Tau pathology and neurodegeneration. Lancet Neurology 12, 609-622). Hyperphosphorylation of tau accounts for its loss of function, gain of toxicity and aggregation to paired helical filaments, which, in turn, form neurofibrillary tangles observed in Alzheimer's disease and several other diseases with tau inclusions (tauopathies) (MG Spillanti, M Goedert (2013) Tau pathology and neurodegeneration. Lancet Neurology 12, 609-622).

Tauopathies include, for example, Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration (some cases caused by C9ORF72 mutations), Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions.

An "effective amount" of a compound, as used herein in the context of an effective amount of said compound being administered to a patient, is an amount of said compound that is capable of achieving the desired increase in the enzymatic activity of protein phosphatase 5, and will vary depending on such factors as age, condition, sex, extent of disease of the patient, counter-indications, if any, and other variables to be adjusted by a physician.

Protein phosphatase 5 (PP5) is a ubiquitously expressed serine/threonine phosphatase that is strictly autoregulated by its tetratricopeptide repeat (TPR) domain and the C-terminal α-helix. Fine-tuning of its dephosphorylation activity modulates a diverse set of cellular key players including pathogenic proteins involved in neurodegenerative disorders. The present inventors have surprisingly found that the compounds according to the present invention are highly selective, allosteric activators of protein phosphatase 5 that enhance the phosphatase activity up to 20-fold without affecting the PP5-Hsp90 interaction. Functional and crystallographic analyses have revealed a relaxation of the autoinhibited state of PP5 upon ligand binding, thus facilitating substrate entry into the phosphatase domain. Application of the small molecule activators of PP5 in a tau-P301L mouse model decreased the pathology-relevant phosphorylation of tau and the degeneration of hippocampal and cortical neurons. Hence, the compounds according to the present invention represent an alternative in the treatment of distinct neurodegenerative disorders, i.e. tauopathies.

Recent studies have suggested that Hsp90 regulates both tau phosphorylation and tau pathology by a co-chaperone complex with PP5 (Salminen et al., 2011). Besides that, tau dephosphorylation by the Hsp90-PP5 complex may ameliorate crucial tau aggregation and facilitate its physiological function including re-binding to the microtubules where PP5 was shown to be localized. Also other protein phosphatases like PP1, PP2A and PP2B may contribute to tau dephosphorylation *in vitro* and *in vivo (Liu et al., 2005α).* PP5 is highly expressed in the mammalian brain, but few physiological substrates have yet been identified (Liu et al., 2005b).PP5 provides a K_{M} (8-13 µM) towards tau in the physiological range of its intraneuronal concentration. Tau is dephosphorylated by PP5 at various Alzheimer's disease (AD)-associated phosphorylation sites (Liu et al., 2005b), including the AT8 epitope (Ser202/Thr205), which we have addressed in our study. Notably, the tau-directed activity of PP5 is significantly decreased in human AD brains (Liu et al., 2005a).

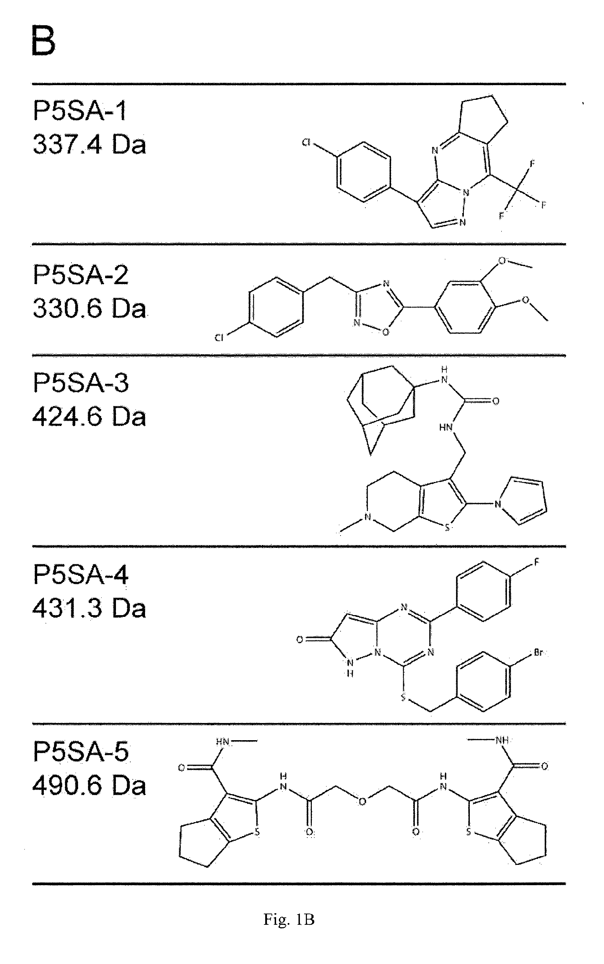

According to the invention, five highly selective PP5 activator classes were identified (termed P5SA-1 to P5SA-5, acronym from *P*rotein phosphatase ***5S***mall molecule *A*ctivator 1 to 5; see Fig. 1B for structure) which showed efficient tau dephosphorylation at the pathology-relevant AT8 epitope (Ser202/Thr205) and were able to decrease human tau-P301L-induced neuronal degeneration in the hippocampal formation of mice.

PP5 is unique in its regulatory mechanism, being the sole enzyme in its class interacting directly with the Hsp90 chaperone system by its TPR domain. The phosphatase activity of PP5 is autoinhibited in the apo-state by a specific interaction between the N-terminal TPR domain and the C-terminal αJ helix which is released upon binding to Hsp90. The complex regulation of PP5 serves to scaffold substrates for dephosphorylation only when recruited to the Hsp90 chaperone network. Notably, the compounds according to the present invention do not interfere with the binding of Hsp90, demonstrating a TPR-independent regulation of the enzymatic activity. This mechanism is distinct from all PP5 activators described so far, including arachidonic acid and its derivatives (Cher et al., 2010; Kang et al., 2001; Ramsey and Chinkers, 2002). Furthermore, the compounds according to the present invention do not share much structural similarity, but despite their heterogeneity, each compound according to the present invention is able to relief the guarded phosphatase domain and increases drastically the turnover rate. Based on deletion constructs and X-ray structures the activator binding region could be allocated to the phosphatase domain at the interface with the TPR domain. At this junction residual electron density was observed only in the PP5:P5SA-2 complex structure implying that this represents the binding cleft for the activator. In contrast to enzyme inhibitors that lock a protein in a specific state, the crystallization of an activated species may render an increase in disorder and flexibility where the activator does not arrest completely and is not fully resolved. The crystallographic results of the PP5-ligand complex further reveal a structural reorganization of the phosphatase's C-terminus and a tilting of single helices of the TPR domain up to 10°. The conformational rearrangements prevent autoinhibition of the phosphatase and open the substrate channel of the enzyme. Hence, the compounds according to the present invention act allosterically and bypass an autoinhibitory mechanism of PP5 that relies on complex protein-protein-interactions in the cellular environment.

The compounds according to the present invention cause PP5 activation by a particularly similar mechanism and affect tau-dependent neuronal degradation. They hold considerable beneficial potential for patients suffering from various neurodegenerative diseases, i.e. tauopathies.

In the following, reference is made to the figures:
All methods mentioned in the figure descriptions below were carried out as described in detail in the examples.

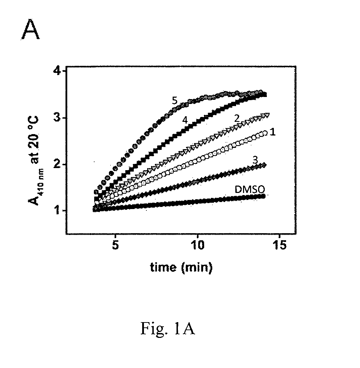

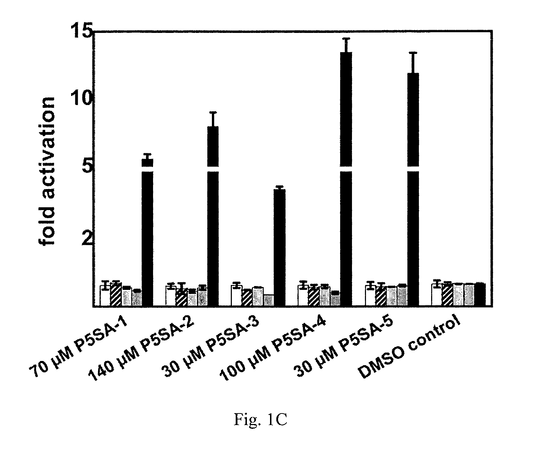

**Figure 1****.** Small molecules specifically activate nematode protein phosphatase 5 (PPH-5).
   (A) A pNPP-based compound screen identified five substances with activating effect on PPH-5, P5SA-1 (yellow, labelled 1), P5SA-2, (orange, labelled 2), P5SA-3, (red, labelled 3), P5SA-4 (blue, labelled 4) and P5SA-5 (green, labelled 5). Concentration of P5SAs in the initial screen was 50 ng/mL. PPH-5 activity in presence of DMSO is depicted in black (labelled "DMSO").
   (B) Chemical structures of the five activators. All compounds were purchased by ChemDiv (San Diego, California, USA).
   (C) The P5SAs are specific for protein phosphatase 5. The human paralogs of PP5, PP1 (white), PP2A (hatched) and PP2B/PP3 (light grey), as well as the bovine intestinal alkaline phosphatase (BIAP, dark grey) are not affected by the small molecules. Each bar represents at least three independent experiments.

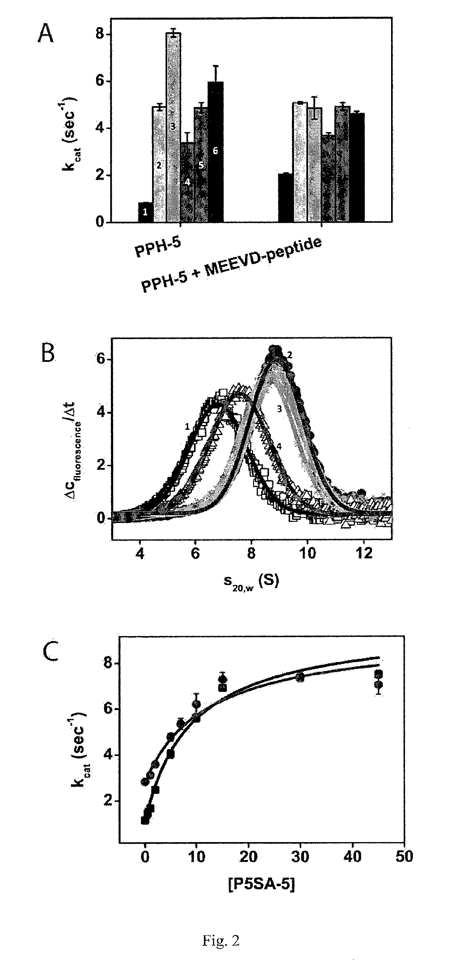

**Figure 2****.** The P5SAs do not interfere with the binding of PPH-5 to the chaperone Hsp90/DAF-21.
   (A) Presence of the C-terminal MEEVD peptide of Hsp90/DAF-21 stimulates the phosphatase activity of PPH-5 around 3-fold (compare black bars, labelled 1). Supplementation with the P5SAs does not lead to additive activation of PPH-5. 70 µM of P5SA-1 (yellow, labelled 2), 140 µM of P5SA-2 (orange, labelled 3), 50 µM of P5SA-3 (red, labelled 4), 100 µM of P5SA-4 (green, labelled 5) and 45 µM of P5SA-5 (blue, labelled 6) were added. Each bar represents at least three independent experiments.
   (B) Complex formation between PPH-5 and Hsp90/DAF-21 is not influenced by the presence of the P5SAs. The sedimentation coefficient of YFP-Hsp90 is shifted from a s_{20,w} value 6.8 S (black, labelled 1) to of 8.9 S in presence of PPH-5 (red, labelled 2). Supplementation of each of the five different P5SAs (orange, labelled 3) does not alter the sedimentation coefficient of the phosphatase-chaperone complex. In contrast, presence of the Hsp90 derived MEEVD peptide destabilizes the complex and shifts the s_{20,w} value from 8.9 S to 7.6 S (green, labelled 4). P5SA-1 and P5SA-2 were added to 70 µM, P5SA-3 to 50 µM, P5SA-4 to 100 µM and 107B3 P5SA-5 to 30 µM.
   (C) Presence of the MEEVD peptide only weakly impacts the binding affinity of P5SA-5 to PPH-5. Titration of PPH-5 with P5SA-5 in absence of MEEVD peptide is depicted as black squares whereas activities in presence of 60 µM peptide are shown as red circles. Each data point represents three independent experiments.

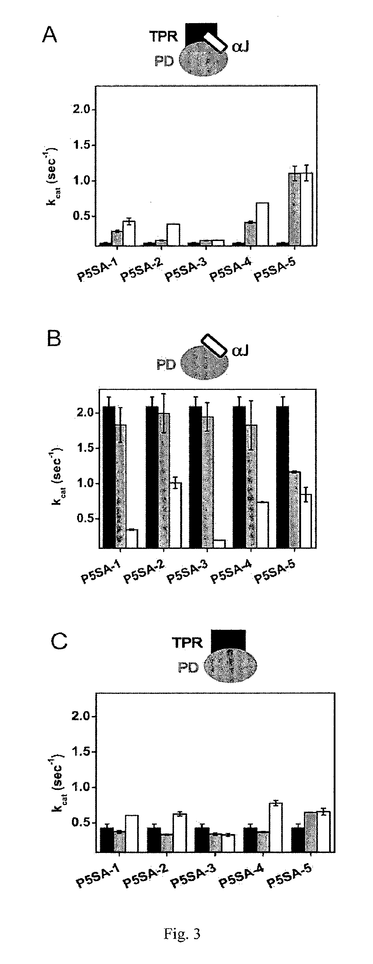

**Figure 3****.** The P5SAs interfere with the autoregulatory mechanisms of mammalian PP5. (A-C) Activity in absence of activators is depicted in black, in presence of 10 µM substance in grey and of 100 µM (45 µM in case of P5SA-5) in white.
   (A) The small molecules activate the full-length protein phosphatase 5.
   (B) The phosphatase domain of PP5 is inhibited by the small molecules demonstrating a direct interaction of the P5SAs with the phosphates domain.
   (C) The PP5 deletion variant PP5-ΔC8 lacks a decisive amino acid stretch of the regulatory αJ domain and shows moderate stimulation by the P5SAs. Activity levels of A, B and C at 100/45 µM of substances (white bars) converge for the different variants of PP5. Each bar represents at least three independent experiments.

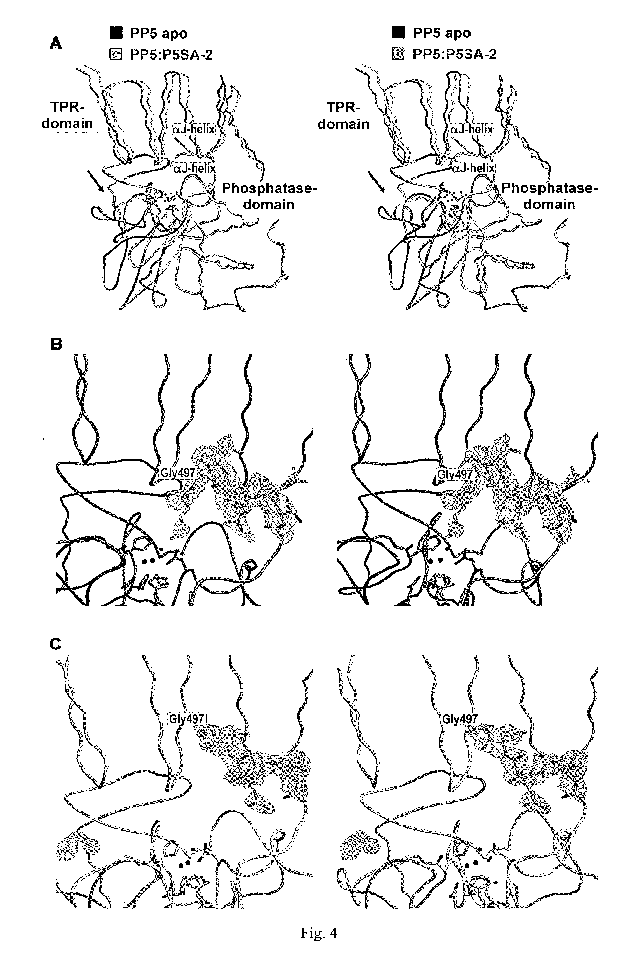

**Figure 4****.** Crystal structures of PP5 both in its apo-state as well as in complex with the ligand P5SA-2.
   (A) Stereo view of the backbone superposition of both molecules shown as ribbon plots. PP5 apo is colored in dark grey with the corresponding αJ-helix colored in green. The PP5:P5SA-2 structure is colored in light grey with the corresponding αJ-helix colored in yellow. The position of the additional electron density, which is only visible in the PP5:P5SA-2 structure is indicated with a red arrow. Active site residues are depicted as sticks and the two magnesium ions (black) and one water molecule (red) are shown as balls.
   (B) Stereo view of the 2Fₒ-F_{c} omit electron density map (countered at 1σ) for the αJ-helix (Thr492 - Met498) of PP5 apo, in which the αJ-helix has been removed for phase calculations. PP5 apo is represented in dark grey coils and amino acid residues of the active site are depicted as sticks. The two magnesium ions as well as a water molecule in the active site are shown as balls. The position of Gly497 is indicated.
   (C) Stereo view of the 2Fₒ-F_{c} omit electron density map (at 1σ) for the αJ-helix (Thr492 - Gly497) of PP5 in complex with P5SA-2. The PP5 backbone is represented in light grey coils.
      The representation of the active site corresponds to (B). The electron density depicted in blue at 1σ indicates the binding site of the ligand P5SA-2.

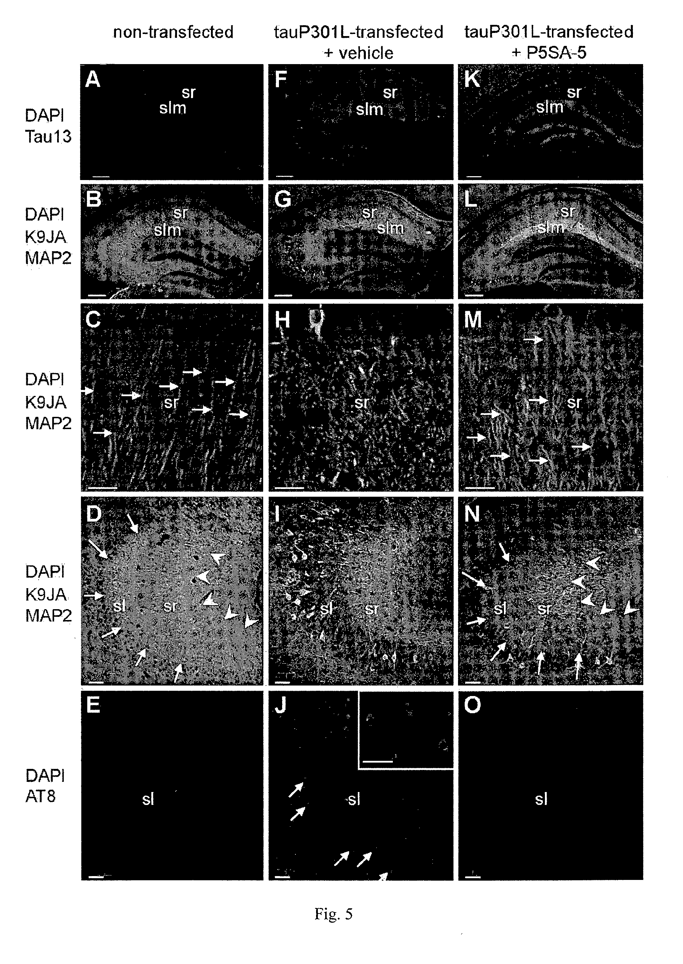

**Figure 5****.** PP5 activation preserves the structural integrity of the hippocampal formation despite human tau-P301L expression.
   (A-E) Non-transfected hippocampi reveal dense neuronal network structures in *Stratum radiatum* (sr) and *Stratum lacunosum moleculare* (slm) of CA1 (B, C) and *Stratum lucidum* (sl) *Stratum radiatum* (sr) of CA3 (B, D).(F-J) After human tau-P301L transfection and vehicle treatment, distinct dendritic (CA1, CA3) and mossy fiber (CA3) degeneration was observed.(K-O) Degeneration was prevented by the PP5 activator P5SA-5 (1 mg/kg/day, continuous ip infusion for six weeks from the time point of tau-P301L transfection until immunohistochemical evaluation). Intact apical dendrites of CA1 pyramidal neurons are marked by arrows (C, M). In CA3 (D, N), the left border of dense sl (green fluorescence) is indicated by arrows and the right border of intact sr (yellow fluorescence) is shown by arrow heads. In contrast to vehicle treatment, phosphorylation of human tau-P301L at its AT8 epitope (Ser202/Thr205) was diminished by P5SA-5 (compare Fig. E, J, O).
   The following markers were used as indicated: DAPI, cell nuclei; tau-13, human tau; K9JA, *total tau;* MAP2, microtubule-associated protein 2; AT8, human phospho-tau (AT8 epitope). For each of the three treatment groups (non-transfected, tau-P301L-transfected + vehicle, tau-P301L-transfected + P5SA-5), representative hippocampal sections, prepared from the same animal, are shown. In total, 3-5 brains per treatment group were analyzed all delivering similar results in condition-specific manner. Only ipsilateral hippocampi are shown. Scale bars = 200 µm (A, B, F, G, K, L) or 50 µm (C-E, H-J, M-O).

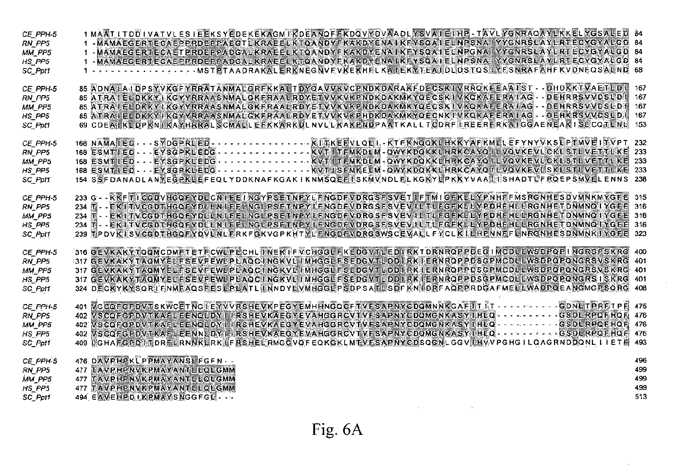

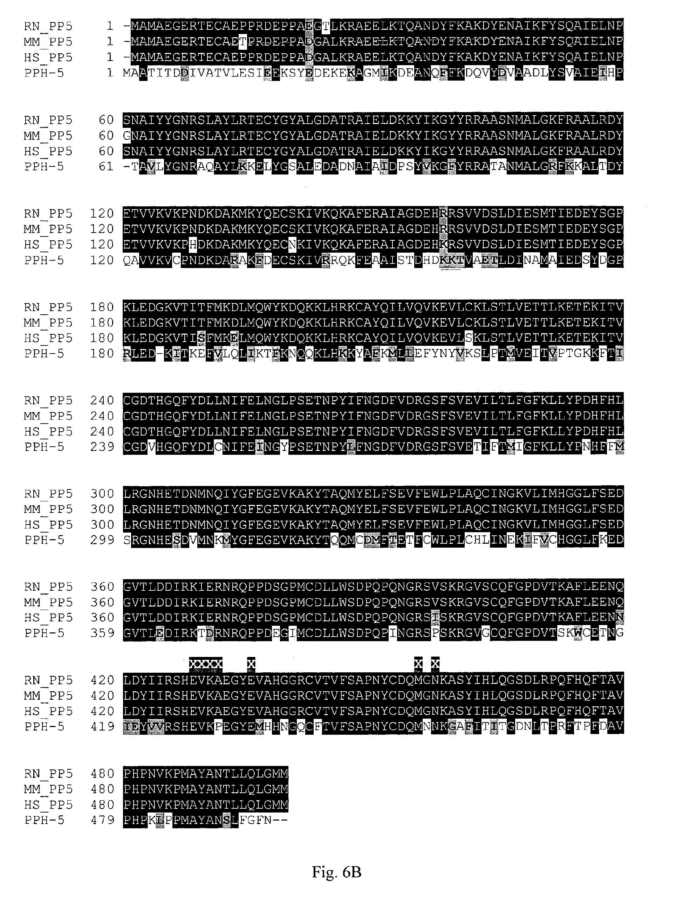

**Figure 6****.** (A) Alignment of protein phosphatase 5 of different species. The protein sequence of PPH-5 of *Caenorhabditis elegans* (CE), PP5 of *Rattus norvegicus* (RN), of *Mus musculus* (MM) and of *Homo sapiens* (HS) and Ppt1 of *Saccharomyces cerevisiae* (SC) were aligned using ClustalW. The alignment was processed by Jalview and therein the Blosum62 matrix used for visualization of the sequence homology.
   (B) Alignment of PPH-5 Homologs. RN_PP: *Rattus norvegicus* protein phosphatase 5, MM_PP5: *Mus musculus* protein phosphatase 5; HS_PP5: *Homo sapiens* protein phosphatase 5; PPH-5: C. *elegans* protein phosphatase 5. Residues which in the electron density map obtained from x-ray crystallography of rat PP5 in complex with P5SA-2 make contact with the small molecule are marked with 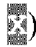 ).

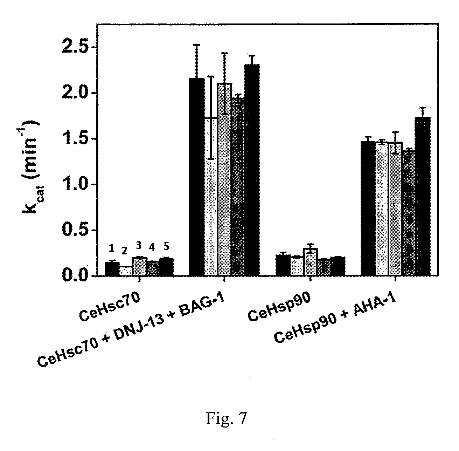

**Figure 7****.** The P5SAs neither influence the ATPase activity of C. *elegans* Hsc70/HSP-1 and *C. elegans* Hsp90/DAF-21 nor interfere with the interaction sites between the chaperones and their cochaperones. Concentrations well above the K_{D} of P5SA-1 (yellow, labelled 2), P5SA-2 (orange, labelled 3), P5SA-4 (red, labelled 4) and P5SA-5 (blue, labelled 5) were tested for their ability to interfere with the ATPase rates of the nematode chaperone systems of Hsc70 and Hsp90. DMSO controls are shown in black (labelled 1). Addition of the nucleotide exchange factor BAG-1 and the J protein DNJ-13 increases the activity of Hsc70/HSP-1, but the P5SAs have no additional effect. The activator of Hsp90 AHA-1 acts as predicted by increasing the ATPase rate. P5SAs have again no effect on this chaperone system. Each bar represents three independent experiments.

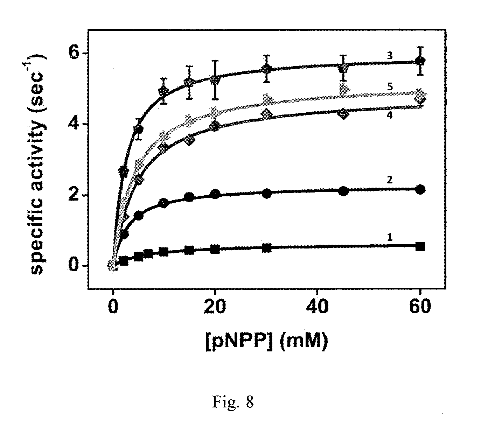

**Figure 8****.** The P5SAs only weakly influence the apparent K_{M} of pNPP. Substrate titrations of pNPP were done with 100 nM of PPH-5. The substrate binding curve in absence of activators is depicted in black (labelled 1). P5SA-1 (blue, labelled 2) was added to 70 µM, P5SA-2 (red, labelled 3) to 140 µM, P5SA-4 (green, labelled 4) to 100 µM and P5SA-5 (orange, labelled 5) to 30 µM. Each data point represents at least three independent experiments.

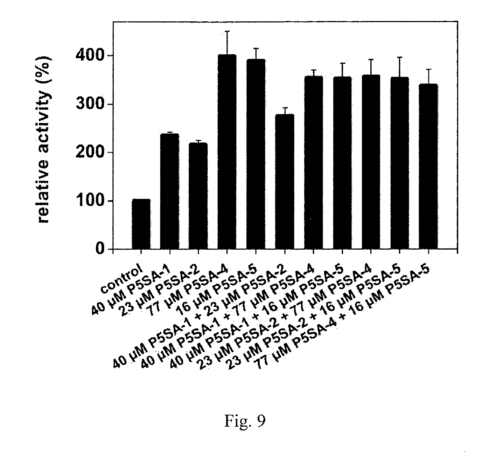

**Figure 9****.** The P5SAs act on the same enzymatic step and are not able to activate in an additive manner. Concentrations of three times the K_{D} were used for each substance and added in different combinations as indicated. Each bar represents three independent experiments.

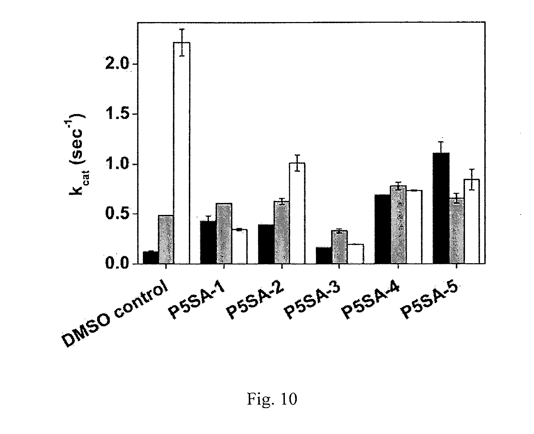

**Figure 10****.** Activity levels of the different deletion constructs of PP5 converge in presence of the P5SAs. Addition of 100 µM of the P5SAs (45 µM in case of P5SA-5) leads to an approximation of the enzymatic activity to similar k_{cat} values of the otherwise different PP5 variants. Full-length PP5 is shown in black, PP5-ΔC8 in grey and PP5-ΔN165 in white where each bar represents three independent experiments.

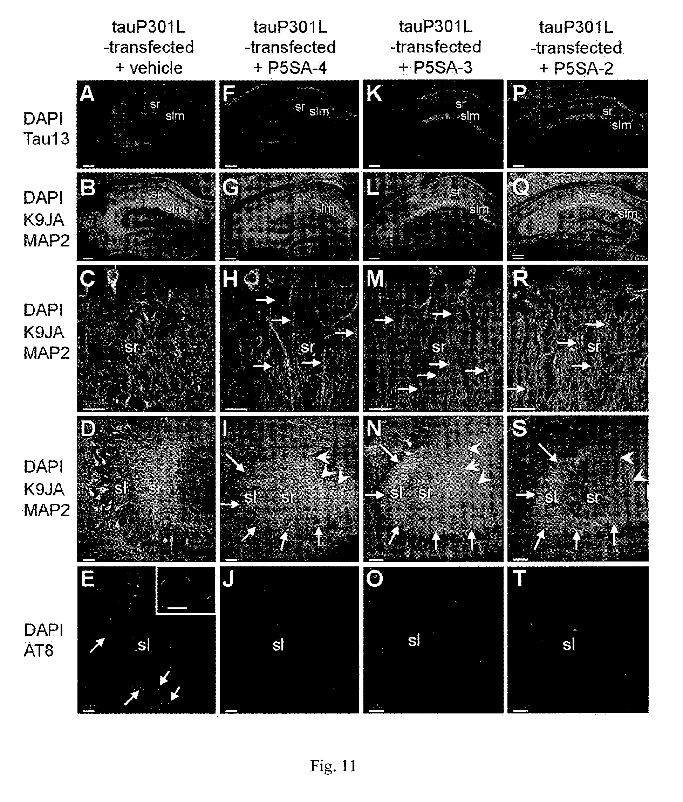

**Figure 11****.** The PP5 activators P5SA-4, P5SA-3 and P5SA-2 induce neuronal protection against human tau-P301L-induced neurodegeneration in the hippocampal formation. (A-E) Human tau-P301L-transfected, vehicle-treated mice brains revealed distinct dendritic (CA1, CA3) and axonal (mossy fibers in CA3) degeneration. Degeneration was particularly visible in Stratum radiatum (sr) and Stratum lacunosum moleculare (slm) of CA1 (B, C) and Stratum lucidum (sl) and Stratum radiatum (sr) of CA3 (B, D). (F-T) This pattern of neuronal degeneration was not observed after administration of PP5 activators P5SA-4, P5SA-3 or P5SA-2 (each drug 1 mg/kg/day, continuous ip infusion for 6 weeks from time point of tau-P301L transfection until immunohistochemical evaluation). Rather, drug-treated hippocampi were characterized by dense neuronal network structures in their CA1 (G, H, L, M, Q, R) and CA3 region (G, I, L, N, Q, S). Intact apical dendrites of CA1 pyramidal neurons are marked by arrows (H, M, R). In CA3 (I, N, S), the left border of dense sl (green fluorescence) is indicated by arrows, whereas the right border of intact sr (yellow fluorescence) is shown by arrow heads. In contrast to vehicle treatment, phosphorylation of human tau-P301L at its AT8 epitope (Ser202/Thr205) was diminished by PP5 activation (compare Figure E, J, O, T).
   The following markers were used as indicated: DAPI, cell nuclei; tau-13, human tau; K9JA,
*total tau;* MAP2, microtubule-associated protein 2; AT8, human phospho-tau (AT8 epitope). For each of the 4 treatment groups (vehicle versus P5SA-4, P5SA-3 or P5SA-2), representative hippocampal sections, prepared from the same animal, are shown. In total, 3-5 brains per treatment group were analyzed, all delivering similar results in condition-specific manner. Only ipsilateral hippocampi are shown. Experiments illustrated in Figure 5 and Figure 11 were performed in parallel. Scale bars = 200 µm (A, B, F, G, K, L, P, Q), 50 µm (D, E, I, J, N, O, S, T) or 20 µm (C, H, M, R).
**Figure 12****.** Amino acid sequences of C. *elegans* protein phosphatase 5 (SEQ ID NO: 1), *Rattus norvegicus* protein phosphatase 5 (SEQ ID NO: 2), *Mus musculus* protein phosphatase 5 (SEQ ID NO: 3) and human protein phosphatase 5 (SEQ ID NO: 4).

In the following, reference is made to the examples, which are given to illustrate, not to limit the present invention.

### Examples

### Example 1

### Materials

Substances P5SA-1, P5SA-2, P5SA-3, P5SA-4 and P5SA-5 (see Fig. 1B for structure) were purchased from ChemDiv (San Diego, California, USA) with the respective ChemDiv-Code 5638-0063, C301-7823, E635-0056, G199-1264 and G839-0123. The C-terminal Hsp90/DAF-21 peptide (AEEDASRMEEVD) was obtained from Biomatik (Cambridge, Ontario, Canada).

### Protein purification

Cloning and purification of PPH-5, Hsp90/DAF-21, CDC-37, YFP-Hsp90/DAF-21 were performed as described before (Gaiser et al., 2009; Gaiser et al., 2011; Gaiser et al., 2010). The purified human phosphatases PP1, PP2A and PP2B/PP3 were purified as described (Baumgrass et al., 2001; Mondragon et al., 1997; Tung et al., 1985). The expression clones of mammalian PP5, PP5-ΔN165 (166-499) and PP5-ΔC8 (1-491) were generated by PCR from plasmid-DNA containing full-length PP5 from *Rattus norvegicus.* PCR-products were inserted into the pET28b expression plasmid (Merck, Darmstadt, Germany) and verified by DNA sequencing (GATC Biotech, Konstanz, Germany). Proteins were expressed in BL21-CodonPlus (DE3)-RIL bacteria (Stratagene, LaJolla, USA). Bacterial cultures were grown to an OD₆₀₀ of 0.8 and induced with 1 mM IPTG. Cells were disrupted using a cell disruption machine (IUL Instruments, Freiburg, Germany) and the lysate was loaded onto a HisTrap FF column (GE Healthcare, Freiburg, Germany). Protein was eluted in a buffer containing 400 mM imidazole. Eluted protein was dialyzed and applied to a ResourceQ anion exchange column (GE Healthcare, Freiburg, Germany). Finally, proteins were subjected to size exclusion chromatography on Superdex 200 PrepGrade gel filtration columns (GE Healthcare, Freiburg, Germany). Protein purity was assessed by SDS-PAGE and the molecular mass was determined by a MALDI-TOF/TOF mass spectrometer (Bruker, Bremen, Germany). Proteins were frozen in liquid nitrogen in 40 mM HEPES/KOH, pH 7.5, 20 mM KCl, 1 mM DTT and stored at -80 °C.

### Phosphatase Assay

Phosphatase activity was followed by the conversion of para-nitrophenyl phosphate (pNPP) to para-nitophenole and absorption of the product at 410 nm (ε: 15100 cm⁻¹ M⁻¹) in a Varian Cary 100 spectrophotometer (Agilent Technologies, Boeblingen, Germany). The reaction was measured in 40 mM HEPES/KOH, 20 mM KCl, 5 mM MnCl₂, 1 mM DTT, pH 7.5 and 60 mM pNPP at 20 °C if not indicated otherwise. Phosphatase concentration ranged from 50 to 500 nM in the assay. If indicated, Hsp90/DAF-21 peptide was supplemented in the assay at a concentration of 60 µM.

### Analytical ultracentrifugation (AUC)

Binding of the P5SAs to the Hsp90/DAF-21 PPH-5 interface was analyzed by analytical ultracentrifugation with fluorescence detection as described before (Gaiser et al., 2010). Experiments were performed in a Beckman ProteomeLab XL-A analytical ultracentrifuge (Beckman, Brea, USA) equipped with an Aviv AU-FDS detector (Aviv Biomedical, Lakewood, USA). AUC runs were performed in 40 mM HEPES/KOH, pH 7.5, 20 mM KCl, 1 mM DTT, the concentration of labeled species was 300 nM and 3 µM of PPH-5. Where indicated 60 µM of the C-terminal Hsp90/DAF-21 peptide (AEEDASRMEEVD) was present in the sample. Centrifugation was performed at 20 °C and 42.000 rpm for 12 hours. Scans were recorded every 90 seconds. Data analysis was performed using a dc/dt approach according to Stafford (Stafford, 1992). The dc/dt plots were fit to Gaussian functions in order to obtain the s_{20,w} values of the respective species as described previously (Gaiser et al., 2011).

### ATPase assay

The activity of the ATPases Hsc70/HSP-1 and Hsp90/DAF-21 were assessed in a coupled regenerative ATPase assay as described before (Gaiser et al., 2009). Reactions were measured in a Varian Cary 100 spectrophotometer (Agilent Technologies, Boeblingen, Germany) at 30 °C and the depletion of NADH was followed at 340 nm. Chaperone concentration was 3 µM and cochaperones were added, when indicated, at a concentration of 5 µM.

### Crystallization and structure determination

PP5 protein was concentrated to 15 mg/mL in the following buffer: 10 mM Tris, pH 7.8, 3 mM DTT. Optionally, the ligand was added with a final concentration of 1 mM. Crystals were grown at 20 °C within 4 weeks by using the hanging drop vapour diffusion method. Drops contained equal volumes of protein and reservoir solutions (0.2 M Mg(NO₃)₂, 20% PEG 3350). Crystals were soaked for 30 seconds in cryo buffer (mother liquor + 25% PEG 200) and were subsequently cooled in liquid nitrogen. Diffraction datasets were recorded using synchrotron radiation of λ = 1.0 Å at the beamline X06SA, Swiss Light Source (SLS), Villigen, Switzerland. Collected datasets were processed using the program package XDS (Kabsch, 1993), statistics are summarized in Table 3.

Determination of the crystal structure was performed by molecular replacement using the program PHASER (McCoy et al., 2007).Human PP5 (PDB ID: 1WAO) was applied as starting model for the ligand structure PP5:P5SA-2. The refined coordinates of PP5:P5SA-2 in turn were employed for the apo-structure of PP5. Model building was carried out with the graphic program MAIN and finalized applying REFMAC5 (Vagin et al., 2004) by conventional crystallographic rigid body, positional, and anisotropic temperature factor refinements with current crystallographic values of *R_{work} =* 23.8%, R_{free} = 27.1%, root mean square deviation (r.m.s.d.) bond lengths of 0.005 Å, and r.m.s.d. bond angle of 0.99° for PP5 apo and *R_{work} =* 21.4 %, R_{free} = 26.1 %, r.m.s.d. bond length of 0.015 Å, and r.m.s.d. bond angle of 1.68° for PP5:P5SA-2.

Coordinates were confirmed to have good stereochemistry indicated by the Ramachandran plot (Table 3). The atomic coordinates have been deposited at the RCSB Protein Data Bank under the accession codes 4JA9 for PP5 apo and 4JA7 for PP5:P5SA-2.

### Antibodies

The following primary antibodies were chosen for immunohistochemical evaluation: Mouse monoclonal anti-human tau-13 (Tau-13, 1:2,000; Covance Research Products Inc., Denver, USA), polyclonal rabbit anti-human tau (K9JA, 1:2,000: Dako, Glostrup, Denmark), mouse monoclonal anti-MAP2 (1:200; Sigma, Saint Louis, USA) and mouse monoclonal anti-human phospho-PHF-Tau (AT8, 1:500; Pierce Biotechnology, Rockford, USA). Alexa Fluor 488 goat anti-rabbit IgG highly cross-adsorbed (1:200; Molecular Probes, Eugene, USA) and Alexa Fluor 594 goat anti-mouse IgG highly cross-adsorbed (1:200; Molecular Probes, Eugene, USA) were used as secondary antibodies.

### Vector and virus construction

The human cDNA *MAPT* variant with no N-terminal inserts and 4 microtubule-binding repeats (0N4R) harboring the point mutation P301L (numbering according to the longest isoform) was amplified by PCR from a vector construct. The PCR construct was cloned into a shuttle vector under control of a CMV promoter and Herpes simplex virus-1 particles were produced by BioVex (Abingdon, UK). Virus titers were 3x10⁹ pfu/mL.

### Animals and stereotactic injection

All protocols for animal experiments described in this study were performed according to the German laws for animal experimentation (Tierschutzgesetz) from 1998. The study was approved by the authorities of the State of Saxony-Anhalt (Landesverwaltungsamt) and performed according to institutional guidelines. Animals were maintained under constant environmental conditions with ambient temperature of 20 ± 2 °C and relative humidity of 50%. They were housed with a 12 hour light-dark cycle and given food and water *ad libitum.* Adult wild-type C57BL/6 mice (10 weeks old) obtained from Charles River Laboratories (Sulzfeld, Germany) were used for all studies. 4% halothane in a mixture of nitrous oxide/oxygen (ratio 70:30) was used for the induction of anesthesia and 1 to 1.5% halothane for the maintenance of anesthesia. Halothane was applied via a nose cone. Throughout all surgical procedures, the animal's body temperature was kept at 37°C using a thermostatically controlled heating blanket. Intracerebral injection of viral particles into the hippocampus of the right brain hemisphere of anesthetized mice was performed stereotactically at coordinates 2.6 mm posterior to bregma, lateral 1.0 mm and ventral 2.0 mm. 2 µl of high titer viral suspension were injected with a fixed syringe (Hamilton Company, Reno, NV, USA). After injection at a rate of 0.2 µl/min, the cannula was left in place for further 30 sec before slow withdrawal. After surgery, the wound was sutured.

### Treatment of P301L transfected mice with PP5 activators via mini-osmotic pumps

Mini-osmotic pumps (Model 2006, Alzet, Palo Alto, CA, USA) were implanted intraperitoneal (ip) through a midline incision immediately after P301L transfection. During implantation, animals remained under halothane anesthesia (details above). The pumps (internal volume: 245.6 µl) were loaded with one of the following substances: P5SA-2, P5SA-3, P5SA-4, P5SA-5 or corresponding vehicle solution (DMSO/polyethylene glycol 400 (50:50 w/w) without drug. P5SA (final concentration 1 mg/kg/day) or vehicle infusion (ip) started immediately after transplantation with a continuous flow rate of 0.15 µl/h and was maintained for 6 weeks.

### Immunohistochemistry

6 weeks after P301L transfection, mice were anesthetized by ip injection of 4% chloral hydrate in PBS, transcardially perfused with ice-cold PBS and fixed with 4% paraformaldehyde in 0.1 M PBS. Immediately after fixation, animals were decapitated. The brains were removed and post-fixed in the same fixative overnight. Fixed brains were placed in a mouse brain matrix (Activational Systems Inc., Warren, USA) and transected into defined coronal brain slices (thickness 1 mm) along the anterior-posterior axis. After incubation in 30% sucrose containing PBS for cryoprotection, 20 µm-thick frontal sections, comprising the hippocampal formation, were cut using a cryostat (Microm International GmbH, Walldorf, Germany). For immunohistochemical staining, sections were washed in 0.1 M PBS. Free-floating sections were permeabilized with 0.2% Triton X-100 in PBS containing 10% normal horse serum for 30 min. After washing with PBS, unspecific antibody binding was blocked by 10% normal horse serum for 30 min, followed by a further wash in 0.1 M PBS. Then, sections were incubated with primary antibodies. For immunofluorescence staining, primary antibodies directed against *total tau* (human plus endogenous mice tau, K9JA) and MAP2 (microtubule-associated protein 2), respectively, were applied simultaneously. Alternatively, the primary antibodies AT8 (human phospho-tau, AT8 (Ser202/Thr205) phosphorylation site) and the human tau-specific primary antibody tau-13 were used individually. After overnight incubation at 4°C, sections were rinsed with 0.1 M PBS. Sections exposed to the primary antibody combination K9JA/MAP2 were incubated with the complementary secondary antibodies goat anti-rabbit IgG Alexa Fluor 488 and goat anti-mouse IgG Alexa Fluor 594. AT8 and tau-13 were visualized with goat anti-mouse IgG Alexa Fluor 594. After final washing in 0.1 M PBS, sections were mounted onto glass slides in Vectashield mounting medium (Vector Laboratories, Burlingame, CA, USA) containing 4',6-diamidino-2-phenylindole (DAPI) nuclear counter stain and cover slipped.

### Microscope image acquisition

An Eclipse TE2000-S inverted fluorescence microscope (Nikon, Düsseldorf, Germany) or the LSM 5 Pascal Exciter laser scanning microscope (Carl Zeiss, Jena, Germany) with the following objectives were used for image acquisition. Eclipse TE2000-S: Plan-Fluor objectives 4x (NA = 0.13) (Nikon, Düsseldorf, Germany), LSM 5 Pascal Exciter: EC Plan-Neofluar Plan-Apochromat 20x (NA = 0.8), EC Plan-Neofluar Plan-Apochromat 40x Oil DIC (NA = 1.30) EC Plan-Neofluar Plan-Apochromat 63x Oil DIC (NA = 1.40) (Carl Zeiss, Jena, Germany). Image acquisition was performed at room temperature. The imaging medium was air, except for the EC Plan-Neofluar 40x and EC Plan-Neofluar 63x objective which required oil immersion. Alexa 594 (red) and Alexa 488 (green) (Molecular Probes, Eugene, OR, USA) coupled to secondary antibodies were used as fluorochromes. A CCD camera (1300QCB, VDS Vosskühler, Osnabrück, Germany) was mounted on the Eclipse TE2000-S and taken for image capture. The Lucia software package (version 4.2.1., Nikon, Düsseldorf, Germany) or LSM 5 Pascal software package (version 4.0, Carl Zeiss, Jena, Germany) were used for setup-specific image acquisition, including brightness and contrast optimization. Brightness and contrast adjustments were applied to each pixel in the image and do not obscure, eliminate or misrepresent any information present in the original, including background. Non-linear adjustments (e.g., changes of gamma settings) were not performed. The graphical layout of all figures was finalized with Microsoft PowerPoint. No specific feature of original images was enhanced, obscured, moved, removed or introduced.

### Statistical analysis of tau phosphorylation

The numbers of AT8-positive perikarya of pyramidal neurons in the hippocampal CA3 region were analyzed using Student's two-sided, heteroscedastic *t* test. Unequal variances were determined with the *f* test (P = 0.145). At least three animals per treatment group and 3 - 5 AT8-stained hippocampal slices per animal were used for analysis. Means ± SEM are given in the text.

### Example 2

### Identification of novel small molecule activators of PPH-5

All methods mentioned in this example were carried out as described in Example 1.

The protein phosphatase 5 is a highly regulated enzyme. The autoinhibition of the phosphatase is controlled by the N-terminal TPR domain and the C-terminal αJ helix, implying a complex mechanism to control the turnover rate (Yang et al., 2005). A compound screen was carried out to identify small molecules which can alter the activity of the phosphatase. The C. *elegans* homolog of protein phosphatase 5 was used (activity of nematode protein phosphatase 5 (PPH-5) at 20 °C: ∼0.62 sec⁻¹; mammalian PP5: ∼0.12 sec⁻¹). The homology between nematode PPH-5 and human PP5 is pronounced with a sequence conservation of almost 80% (Figure 6). Using the substrate para-nitrophenyl phosphate (pNPP), a library of 15,000 compounds was analyzed. While most of these compounds did not affect the enzymatic activity of PPH-5 (see Table 1 for examples of compounds that had no effect on the enzymatic activity of PPH-5), five substances were identified which strongly accelerate the enzymatic activity of PPH-5 (Figure 1A). All of these molecules found to accelerate the enzymatic activity of PPH-5 are smaller than 500 Da (Figure 1B), are stable at room temperature and follow the Lipinsky's rule of five. For convenience, these substances are referred to with the acronym ***P***rotein phosphatase ***5S***mall molecule ***A***ctivator 1 to 5 (P5SA-1 to P5SA-5). The P5SAs were utilized at different concentrations to determine the apparent K_{D}-value in the pNPP-based assay yielding dissociation constants in the range of 6 µM to 26 µM causing a 4 to 10-fold activation under these conditions (Table 2).

**Table 1. Examples for compounds tested that had no effect on nematode PPH-5.**

| Nummer | Struktur |
|---|---|
| G008-3258 | 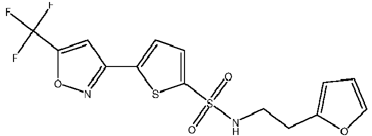 |
| D087-0541 | 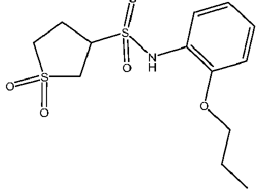 |
| 8016-8528 | 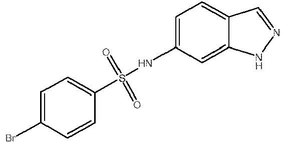 |
| G006-0077 | 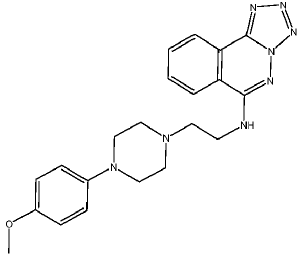 |
| 7896-0152 | 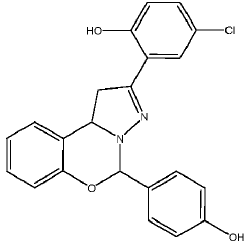 |
| E959-0899 | 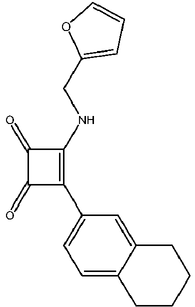 |
| 6125-0074 | 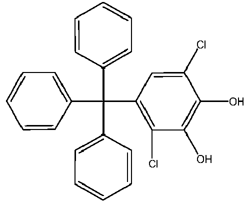 |
| 4327-3176 | 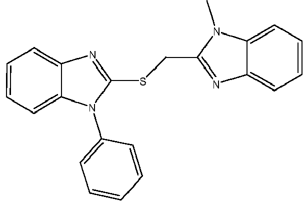 |
| 0760-0044 | 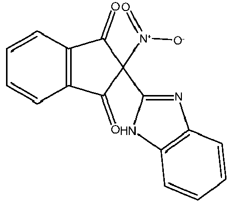 |
| D264-0030 | 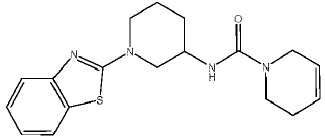 |
| E966-0136 | 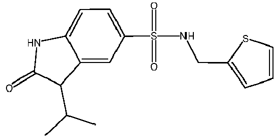 |
| C679-2579 | 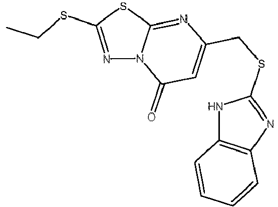 |
| E975-1466 | 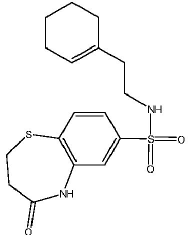 |
| 8015-7443 | 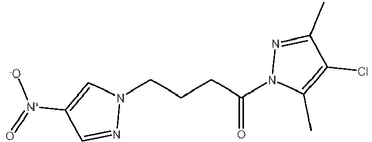 |
| G023-0729 | 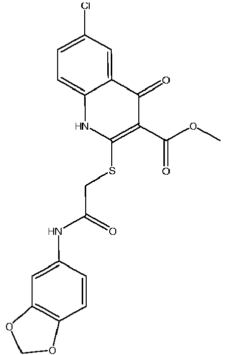 |
| 8001-5069 | 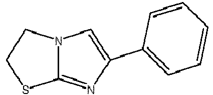 |

**Table 2. The enzymatic parameters of nematode PPH-5 and mammalian PP5 are influenced by the P5SAs.**

| | **PPH-5 (C. *elegans)*** | | | | | **PP5** (***R**. **norvegicus)*** | |
|---|---|---|---|---|---|---|---|
| | **K_{D} (µM)** | **K_{M} (mM)** | **k_{cat} (sec⁻¹)** | **fold activation** | | **fold activation** | |
| **DMSO** | - | 7.0±0.4 | 0.62±0.02 | - | | - | |
| **P5SA-1** | 13.3 ± 4.2 | 3.0 ± 0.2 | 2.28 ± 0.03 | 3.7 | (sat.) | 2.7 | at 143 µM |
| **P5SA-2** | 7.8 ± 1.4 | 2.5 ± 0.1 | 5.98 ± 0.06 | 9.6 | (sat.) | 13.7 | at 714 µM |
| **P5SA-3** | N/D | N/D | N/D | 4.0 | at 714 µM | 7.0 | at 714 µM |
| **P5SA-4** | 25.7 ± 5.5 | 5.0 ± 0.3 | 4.85 ± 0.08 | 7.8 | (sat.) | 16.4 | at 143 µM |
| **P5SA-5** | 6.4 ± 1.8 | 4.3 ± 0.3 | 5.22 ± 0.09 | 8.4 | (sat.) | 22.1 | at 45 µM |

### Example 3

### Small molecule activation is highly specific to PP5-like proteins

All methods mentioned in this example were carried out as described in Example 1.

In this Example, the specificity of the identified activators was studied. First, it was tested whether other enzyme classes are influenced by the presence of the small molecules. To check if the P5SAs show unspecific effects on other proteins, the ATPase active Hsc70 and Hsp90 were employed. As these chaperones potentially bind via hydrophobic patches to a broad set of proteins and nonpolar structures, it seemed to be a well-suited control for unspecific binding of the screened molecules. The P5SAs neither impacted the ATPase activity of both chaperones, nor abrogated the binding of cofactors to Hsc70 or Hsp90 (Figure 7).

Since the phosphatase domain of members of the PPP family is highly homologous, human PP1, PP2A and PP2B/PP3 were analyzed: neither activation nor inhibition of the respective phosphatase activity was detected, implying that the identified molecules are specific activators of the Hsp90-associated PP5/PPH-5 (Figure 1C). Unspecific pNPP hydrolysis could be excluded as well, as bovine intestinal alkaline phosphatase (BIAP) was not affected. The next step was to challenge homologs of PPH-5 from other species. No activation of the yeast homolog Ppt1 which shows reduced similarity of around 60% and more sequence deviations to human PP5, was detected (Figure 6). However, PP5 from *Rattus norvegicus* with 98% sequence identity to human PP5 exhibited vast activation when treated with P5SAs and showed an even more pronounced response than nematode PPH-5 used in the initial screen (Table 2). Therefore the affinity of these activators towards the mammalian protein was determined, resulting in a binding constant of 10 ± 5 µM for P5SA-5, while the other P5SAs showed decreased affinities towards rat PP5 in comparison to PPH-5 from C. *elegans* (Table 2). The conserved activation potential of the P5SAs for higher eukaryotes and their selectivity for PP5 suggest that the activation depends on the specific architecture of PP5 and its complex reaction pathway.

### Example 4

### The PSSAs are allosteric regulators of PPH-5

All methods mentioned in this example were carried out as described in Example 1.

In order to gain insight into the activation mechanism, it was analyzed whether the substrate pNPP interacts with the P5SAs during the dephosphorylation reaction. Direct involvement of the small molecules in the hydrolysis of pNPP should be reflected in a change of the apparent K_{M} value under saturating concentrations of the activators. All P5SAs reflect a strong alteration of the k_{cat} value, but only weakly influence the observed K_{M}-value for pNPP (Figure 8, Table 2). Hence, there is no specific interaction between the substrate pNPP and the P5SAs demonstrating allosteric activation of PPH-5.

Given that all five P5SAs are activators of PPH-5, the question arose whether they stimulate in an additive manner. To address this, the combinatorial effects of the P5SAs on the stimulation of pNPP hydrolysis were measured. Substance concentrations of 3 times higher than the apparent K_{D} value were used by combining the different activators. The observed activities showed no additional stimulation and complied with the estimations of individual but competitive interaction (Figure 9). These results suggest that the P5SAs accelerate the same rate-limiting step during the enzymatic cycle.

### Example 5

### Hsp90 binding is not compromised by the small molecules

All methods mentioned in this example were carried out as described in Example 1.

Human PP5 is well-known to be activated upon binding to the molecular chaperone Hsp90 or C-terminal fragments thereof (Yang et al., 2005). The addition of the Hsp90-derived C-terminal MEEVD peptide resulted in activation of the nematode PPH-5 (Figure 2A). The proposed mechanism implies an autoinhibitory interaction between the binding groove of the TPR domain and the C-terminal αJ helix of the phosphatase domain, which is maintained until the TPR domain is occupied by the MEEVD-motif.

Interestingly, the Hsp90 peptide has only limited effect when supplemented in the presence of the P5SAs (Figure 2A). These findings indicate that the same regulatory mechanism is targeted by both, the small molecules and the MEEVD-motif of Hsp90, which causes the breakup of the autoinhibition. Consequently, it was analyzed whether the P5SAs exclude the binding of Hsp90 to the TPR domain. N-terminal YFP-tagged Hsp90 was used in sedimentation velocity ultracentrifugation experiments. By this technique complexes with PPH-5 induce a shift of YFP-Hsp90's sedimentation coefficient from 6.8 S to 8.9 S (Figure 2B). Binding of TPR cofactor and chaperone was analyzed in the presence of the P5SAs. None of the P5SAs abrogated the complex formation, while the isolated MEEVD peptide clearly weakened the protein complex between Hsp90 and PPH-5.

It was further tested whether P5SA affinity is affected in the presence of large excess of MEEVD peptide. 60 µM of the peptide were supplemented and the binding probability of P5SA-5 was evaluated by the phosphatase assay. Only a minor influence on its affinity was observed despite the high concentration of MEEVD peptide (Figure 2C).

Thus, the P5SAs appear to bind to a site distinct from the peptide binding groove of the TPR domain while releasing autoinhibition similar to the Hsp90 derived peptide.

### Example 6

### The P5SAs bind at the intersection of the phosphatase and the TPR domain

All methods mentioned in this example were carried out as described in Example 1.

For characterization of the binding site of the P5SAs, deletion constructs of rat PP5 were generated. Using the mammalian homolog of the protein phosphatase 5 enabled to study the impact of certain domains of the phosphatase, as shortened variants of C. *elegans* PPH-5 resulted in insoluble protein. The full-length PP5 and its deletion variants were purified and the activity was determined in the absence and presence of 10 µM and 100 µM (45 µM in case of P5SA-5) of the substances, respectively (Figure 3A-C).

The activity of the phosphatase domain variant PP5-ΔN165 which lacks the complete TPR domain and is therefore not autoinhibited, was increased almost 20-fold from 0.12 sec⁻¹ to 2.22 sec⁻¹ compared to wild type protein. Remarkably, while wt-PP5 is strongly activated by the P5SAs, the P5SAs are able to inhibit the phosphatase activity of the TPR domain free protein (Figure 3B). These results point out that the P5SAs do not bind to the TPR domain of PP5, but interact with the phosphatase domain.

Next the second functional region of the autoinhibitory mechanism, the αJ helix, was analyzed. The last 8 amino acids of the protein were deleted including Q495 which is known to be essential for maintaining the tight regulation of PP5 (Kang et al., 2001). Furthermore, the autoinhibitory mechanism of PP5-ΔC8 was disturbed causing an increased turnover of 0.49 sec⁻¹ compared to full-length PP5. Interestingly, addition of the P5SAs still influenced the activity of PP5-ΔC8, but only with a minor effect (Figure 3C, see crystallographic results below).

Based on these findings, the P5SAs do not appear to interact directly with the autoregulatory domains. Instead, the modulators bind to the phosphatase core domain which appears sufficient to control the turnover rate independent of the PP5 variant used in this study (Figure 10).

Therefore the crystal structures of rat PP5 in its apo-state as well as in complex with the ligand P5SA-2 were determined. The structures were solved by molecular replacement (McCoy et al., 2007) using the atomic coordinates of human PP5 (PDB ID: 1WAO) as a search model. The PP5:P5SA-2 dataset was refined to 2.0 Å (R_{free} = 26.1%; PDB ID: 4JA7) and used as a model to determine the apo-structure with a final resolution of 2.3 Å (R_{free} = 27.1%; PDB ID: 4JA9, Table 3).

**Table 3. Data collection and refinement statistics**

| | **PP5 apo** | **PP5** + **P5SA-2** |
|---|---|---|
| ***Crystal parameters*** | | |
| **Space group** | P4₁2₁2 | P4₁2₁2 |
| **Cell constants** | a=b=50.1Å, c=379.2Å | a=b=51.1Å, c=365.7Å |
| **Molecules per AU^{a}** | 1 | 1 |

| ***Data collection*** | | |
|---|---|---|
| **Beamline** | SLS, PXI - X06SA | SLS, PXI - X06SA |
| **Wavelength (Å)** | 1.0 | 1.0 |
| **Resolution range (Å)^{b}** | 40 - 2.3 | 30 - 2.0 |
| **No. observed reflections** | 140950 | 286970 |
| **No. unique reflections^{c}** | 22932 | 34075 |
| **Completeness (%)^{b}** | 99.5 (99.9) | 98.5 (93.1) |
| **R_{merge} (%)^{b,d}** | 4.1 (53.7) | 3.8 (43.5) |
| **I/σ(I)^{b}** | 22.3 (4.4) | 28.8 (5.6) |

| ***Refinement (REFMAC5)*** | | |
|---|---|---|
| **Resolution range (Å)** | 15 - 2.3 | 15 - 2.0 |
| **No. atoms** | | |
| **Protein** | 3757 | 3749 |
| **Water** | 63 | 106 |
| **R_{work/}R_{free} (%)^{e}** | 23.8 / 27.1 | 21.4 / 26.1 |
| **r.m.s. deviations^{f}** | | |
| **bond lengths/angles (Å)/(°)** | 0.005 / 0.99 | 0.015 / 1.68 |
| **Average B-factor (Å²)** | 74.1 | 51.0 |
| **Ramachandran plot (%)^{g}** | 95.2 / 4.6 / 0.2 | 95.7 / 4.3 / 0 |
| **PDB accession code** | 4JA9 | 4JA7 |

| | | |
|---|---|---|
| ^{a} Asymmetric unit ^{b} The values in parentheses of resolution range, completeness, R_{merge} and I/σ (I) correspond to the last resolution shell. ^{c} Friedel pairs were treated as identical reflections. ^{d}R_{merge}(I) = ∑ₕₖₗ∑ⱼ\|[I(*hkl*)ⱼ-I(*hkl*)]\|/[∑ₕₖₗIₕₖₗ], where I(hkl)ⱼ is the j^{th} measurement of the intensity of reflection hkl and <I(hkl)> is the average intensity. ^{e} R = ∑ₕₖₗ\| \|F_{obs}\| - \|F_{calc}\| \|/∑ₕₖₗ \|F_{obs}\|, where R_{free} is calculated without a sigma cutoff for a randomly chosen 5% of reflections, which were not used for structure refinement, and R_{work} is calculated for the remaining reflections. ^{f} Deviations from ideal bond lengths/angles ^{g} Number of residues in favoured region / allowed region / outlier region | | |

In both structures the first defined amino acid is Gly23 and the loop connecting the TPR domain with the phosphatase domain (Arg150 - Arg158; numbers according to full-length ratPP5) are flexible and therefore not defined in the electron density map. Additionally, Met499 of the apo-structure and Met498 as well as Met499 of the active ligand structure were not resolved. The electron density of P5SA-2 is only partially defined, however, comparison of both structures, PP5 apo and the PP5:P5SA-2 complex, clearly assigned the binding site of the ligand at the interface between the TPR and the phosphatase domain.

A comparison of the backbone traces discloses limited structural rearrangements within the phosphatase domain, reflected by a r.m.s. deviation of the Cα-atoms of 0.42 Å (Figure 4A). By contrast, a tilting of the TPR domain up to 10° is observed upon ligand binding (r.m.s. deviation: 0.84 Å) accompanied by a rearrangement of the αJ helix. These structural domain reorganizations are manifested in a reduction of the crystal lattice constant c by 15 Å. Inspecting the omit maps for both αJ helices ranging from Asn491 to Gly497 or to Met498, respectively, a considerable displacement of the Cα-atom of Gly497 by 6.5 Å is observed (Fig 5B and 5C). The conformational rearrangements of both the TPR domain and the αJ helix indicates opening of the active site channel as displayed by the *in silico* values for the surface area and the volume of the substrate binding pocket. In the apo-state the binding pocket covers approximately 1250 Å² with a corresponding volume of 1710 Å² (Dundas et al., 2006), whereas in the active state both values increase significantly (1370 Å² and 2090 Å²), suggesting facilitated substrate access into and product release from the active site channel, respectively. In summary, the observed structural rearrangements induced by ligand binding support the functional characterization of the P5SAs that act on the release of the autoinhibiton of PP5.

### Example 7

### PP5 activation decreases pathological tau phosphorylation and prevents human tau P301L-induced neuronal degeneration in the hippocampus

All methods mentioned in this example were carried out as described in Example 1.

The intracellular activity of the identified activators was addressed by analyzing their physiological effects *in vivo.* Therefore an experimental model was chosen, in which PP5 activation induces beneficial effects. The human tau variant tau-P301L is a key mutant in familial frontotemporal dementia with Parkinsonism linked to chromosome 17 (FTDP-17). Tau-P301L is able to interact with wild-type tau and to seed a tau-driven pathogenesis under experimental conditions. Hence, the influence of P5SA-2, P5SA-3, P5SA-4 and P5SA-5 on tau phosphorylation and tau-induced pathogenesis in human tau-P301L-transfected mice brains was analyzed.

Adult wild-type C57BL/6 mice brains were transfected with a Herpes simplex-derived viral vector construct containing human tau-P301L by unilateral vector injection into the hippocampal CA1 region adjacent to the subiculum. In non-transfected control mice, endogenous tau was detectable in a pronounced manner in axons of granule cells of the dentate gyrus (mossy fibers), projecting to apical dendrites of CA3 pyramidal neurons (Stratum lucidum) (Figure 5B-D). Endogenous tau was also present in apical dendrites of pyramidal CA1 (stratum radiatum, stratum lacunosum moleculare) (Figure 5B,C) and CA3 neurons (stratum radiatum) (Figure 5B-D), whereas it was absent in neuronal perikarya of CA1/CA3 pyramidal cells and granular cells of dentate gyrus (Figure 5B-D). Endogenous mouse and/or transfected human tau-P301L were collectively visualized as *total tau* with the antibody K9JA which does not discriminate between mouse and human. Viral vector-based human tau-P301L gene transfection resulted in its abundant and neuron-specific expression in the hippocampus six weeks post transfection, as visualized by the human tau-specific antibody tau-13 (compare Figure 5A,F,K). In transfected, but otherwise vehicle-treated animals, human tau-specific fluorescence staining (tau-13) was particularly found in the perikarya and apical dendrites of CA1 and CA3 pyramidal neurons (Figure 5F). In contrast to non-transfected animals, tau-P301L expression in transfected mice was accompanied by the increase of *total tau* in the perikarya of CA1 and CA3 pyramidal neurons (Figure 5G-I). In parallel to human tau-P301L expression, its phosphorylation at the AT8 epitope (Ser202/Thr205) was observed (Figure 5J). Whereas non-transfected hippocampi were characterized by a dense and intact structural integrity throughout the CA1 and CA3 region (Figure 5B-D), their human tau-P301L-transfected counterparts revealed a massive destruction in hippocampal CA1 (stratum radiatum, stratum lacunosum moleculare) (Figure 5G,H) and CA3 region (stratum lucidum, stratum radiatum) (Figure 5G,I). As visualized by MAP2- and *total tau*-specific immunofluorescence, the defined loss of structural integrity was caused by the degeneration of apical dendrites of CA1 and CA3 pyramidal neurons and axonal mossy fibers of dentate gyrus granule cells (compare Figure 5B-D and 5G-I). This tau-P301L-dependent pattern of neuronal degeneration was decreased, if the PP5 activator P5SA-5 was administered (Figure 5K-N). Continuous drug infusion (ip) was started immediately after tau-P301L transfection and maintained over six weeks until immunohistochemical evaluation (final drug concentration 1 mg/kg/day). In parallel to the preservation of apical CA1/CA3 dendrites and mossy fibers, pathological tau phosphorylation at the AT8 epitope was remarkably reduced by P5SA-5 treatment (Figure 5J,O). Per hippocampal slice, the numbers of AT8-positive perikarya of CA3 pyramidal neurons were 7.0 ± 2.0 and 2.2 ± 1.4 in vehicle- and P5SA-5-treated animals, respectively (*P*< 0.0001). In parallel, tau-P301L-dependent localization of *total tau* signals in the perikarya of CA1 and CA3 pyramidal neurons remained unaffected (Figure 5L-N).

Similar observations were made with P5SA-2, P5SA-3 and P5SA-4 (Figure 11).

The features of the present invention disclosed in the specification, the claims, and/or in the accompanying drawings may, both separately and in any combination thereof, be material for realizing the invention in various forms thereof.

### References

Bahl, R., Bradley, K.C., Thompson, K.J., Swain, R.A., Rossie, S., and Meisel, R.L. (2001). Localization of protein Ser/Thr phosphatase 5 in rat brain. Brain research Molecular brain research 90, 101-109.
Baumgrass, R., Weiwad, M., Erdmann, F., Liu, J.O., Wunderlich, D., Grabley, S., and Fischer, G. (2001). Reversible inhibition of calcineurin by the polyphenolic aldehyde gossypol. Journal of Biological Chemistry 276, 47914-47921.
Brunden, K.R., Trojanowski, J.Q., and Lee, V.M. (2009). Advances in tau-focused drug discovery for Alzheimer's disease and related tauopathies. Nature reviews Drug discovery 8, 783-793.
Chen, M.X., McPartlin, A.E., Brown, L., Chen, Y.H., Barker, H.M., and Cohen, P.T. (1994). A novel human protein serine/threonine phosphatase, which possesses four tetratricopeptide repeat motifs and localizes to the nucleus. The EMBO journal 13, 4278-4290.
Cher, C., Tremblay, M.H., Barber, J.R., Chung Ng, S., and Zhang, B. (2010). Identification of chaulmoogric acid as a small molecule activator of protein phosphatase 5. Applied biochemistry and biotechnology 160, 1450-1459.
Cliff, M.J., Harris, R., Barford, D., Ladbury, J.E., and Williams, M.A. (2006). Conformational diversity in the TPR domain-mediated interaction of protein phosphatase 5 with Hsp90. Structure 14, 415-426.
Dundas, J., Ouyang, Z., Tseng, J., Binkowski, A., Turpaz, Y., and Liang, J. (2006). CASTp: computed atlas of surface topography of proteins with structural and topographical mapping of functionally annotated residues. Nucleic acids research 34, W116-118.
Gaiser, A.M., Brandt, F., and Richter, K. (2009). The non-canonical Hop protein from Caenorhabditis elegans exerts essential functions and forms binary complexes with either Hsc70 or Hsp90. Journal of molecular biology 391, 621-634.
Gaiser, A.M., Kaiser, C.J., Haslbeck, V., and Richter, K. (2011). Downregulation of the Hsp90 system causes defects in muscle cells of Caenorhabditis elegans. PloS one 6, e25485. Gaiser, A.M., Kretzschmar, A., and Richter, K. (2010). Cdc37-Hsp90 complexes are responsive to nucleotide-induced conformational changes and binding of further cofactors. The Journal of biological chemistry 285, 40921-40932.
Haass, C. (2010). Initiation and propagation of neurodegeneration. Nature medicine 16, 1201-1204.
Hajduk, P.J., Olejniczak, E.T., and Fesik, S.W. (1997). One-dimensional relaxation- and diffusion-edited NMR methods for screening compounds that bind to macromolecules. J Am Chem Soc 119, 12257-12261.
Jack, C.R., Jr., Knopman, D.S., Jagust, W.J., Petersen, R.C., Weiner, M.W., Aisen, P.S., Shaw, L.M., Vemuri, P., Wiste, H.J., Weigand, S.D., et al. (2013). Tracking pathophysiological processes in Alzheimer's disease: an updated hypothetical model of dynamic biomarkers. Lancet neurology 12, 207-216.
Jeganathan, S., Hascher, A., Chinnathambi, S., Biernat, J., Mandelkow, E.M., and Mandelkow, E. (2008). Proline-directed pseudo-phosphorylation at AT8 and PHF1 epitopes induces a compaction of the paperclip folding of Tau and generates a pathological (MC-1) conformation. The Journal of biological chemistry 283, 32066-32076.
Kabsch, W. (1993). Automatic Processing of Rotation Diffraction Data from Crystals of Initially Unknown Symmetry and Cell Constants. Journal of applied crystallography 26, 795-800.
Kang, H., Sayner, S.L., Gross, K.L., Russell, L.C., and Chinkers, M. (2001). Identification of amino acids in the tetratricopeptide repeat and C-terminal domains of protein phosphatase 5 involved in autoinhibition and lipid activation. Biochemistry 40, 10485-10490.
Li, X., Kumar, Y., Zempel, H., Mandelkow, E.M., Biernat, J., and Mandelkow, E. (2011). Novel diffusion barrier for axonal retention of Tau in neurons and its failure in neurodegeneration. The EMBO journal 30, 4825-4837.
Liu, F., Grundke-Iqbal, I., Iqbal, K., and Gong, C.X. (2005a). Contributions of protein phosphatases PP1, PP2A, PP2B and PP5 to the regulation of tau phosphorylation. The European journal of neuroscience 22, 1942-1950.
Liu, F., Iqbal, K., Grundke-Iqbal, I., Rossie, S., and Gong, C.X. (2005b). Dephosphorylation of tau by protein phosphatase 5: impairment in Alzheimer's disease. The Journal of biological chemistry 280, 1790-1796.
McConnell, J.L., and Wadzinski, B.E. (2009). Targeting protein serine/threonine phosphatases for drug development. Molecular pharmacology 75, 1249-1261.
McCoy, A.J., Grosse-Kunstleve, R.W., Adams, P.D., Winn, M.D., Storoni, L.C., and Read, R.J. (2007). Phaser crystallographic software. Journal of applied crystallography 40, 658-674.
Mondragon, A., Griffith, E.C., Sun, L., Xiong, F., Armstrong, C., and Liu, J.O. (1997). Overexpression and purification of human calcineurin alpha from Escherichia coli and assessment of catalytic functions of residues surrounding the binuclear metal center. Biochemistry 36, 4934-4942.
Ramsey, A.J., and Chinkers, M. (2002). Identification of potential physiological activators of protein phosphatase 5. Biochemistry 41, 5625-5632.
Salminen, A., Ojala, J., Kaarniranta, K., Hiltunen, M., and Soininen, H. (2011). Hsp90 regulates tau pathology through co-chaperone complexes in Alzheimer's disease. Progress in neurobiology 93, 99-110.
Sklenar, V., Piotto, M., Leppik, R., and Saudek, V. (1993). Gradient-Tailored Water Suppression for H-1-N-15 Hsqc Experiments Optimized to Retain Full Sensitivity. J Magn Reson Ser A 102, 241-245.
Spillanti, M.G., and Goedert, M. (2013) Tau pathology and neurodegeneration. Lancet Neurology 12, 609-622
Stafford, W.F., 3rd (1992). Boundary analysis in sedimentation transport experiments: a procedure for obtaining sedimentation coefficient distributions using the time derivative of the concentration profile. Analytical biochemistry 203, 295-301.
Tung, H.Y., Alemany, S., and Cohen, P. (1985). The protein phosphatases involved in cellular regulation. 2. Purification, subunit structure and properties of protein phosphatases-2A0, 2A1, and 2A2 from rabbit skeletal muscle. European journal of biochemistry / FEBS 148, 253-263.
Vagin, A.A., Steiner, R.A., Lebedev, A.A., Potterton, L., McNicholas, S., Long, F., and Murshudov, G.N. (2004). REFMAC5 dictionary: organization of prior chemical knowledge and guidelines for its use. Acta crystallographica Section D, Biological crystallography 60, 2184-2195.
Yang, J., Roe, S.M., Cliff, M.J., Williams, M.A., Ladbury, J.E., Cohen, P.T., and Barford, D. (2005). Molecular basis for TPR domain-mediated regulation of protein phosphatase 5. The EMBO journal 24, 1-10.

## Claims

1. A compound that shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
wherein a result is considered a positive result if the enzymatic activity of protein phosphatase 5 in the presence of the compound to be tested is by at least a factor of 1.5 higher than in the absence of the compound to be tested,
wherein said compound is a small molecule compound,
and wherein said compound is not a fatty acid or a derivative thereof.

2. A compound that binds in an *in vitro* binding assay to protein phosphatase 5 within the region of amino acids 166 to 496 of the *C*. *elegans* protein phosphatase 5 amino acid sequence or within the region of amino acids 166 to 499 of the rat protein phosphatase 5 amino acid sequence or within the region of amino acids 166 to 499 of the mouse protein phosphatase 5 amino acid sequence or within the region of amino acids 166 to 499 of the human protein phosphatase 5 amino acid sequence,
wherein said compound is a small molecule compound,
and wherein said compound is not a fatty acid or a derivative thereof.

3. A compound that shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
wherein a result is considered a positive result if the enzymatic activity of protein phosphatase 5 in the presence of the compound to be tested is by at least a factor of 1.5 higher than in the absence of the compound to be tested,
wherein said compound binds in an *in vitro* binding assay to protein phosphatase 5 within the region of amino acids 166 to 496 of the *C*. *elegans* protein phosphatase 5 amino acid sequence or within the region of amino acids 166 to 499 of the mammalian protein phosphatase 5 amino acid sequence or within the region of amino acids 166 to 499 of the rat protein phosphatase 5 amino acid sequence or within the region of amino acids 166 to 499 of the mouse protein phosphatase 5 amino acid sequence or within the region of amino acids 166 to 499 of the human protein phosphatase 5 amino acid sequence,
wherein said compound is a small molecule compound,
and wherein said compound is not a fatty acid or a derivative thereof.

4. A method of screening for compounds that activate protein phosphatase 5, said method comprising
- providing a small molecule compound that is not a fatty acid or a derivative thereof,
- determining whether said compound shows a positive result in a phosphatase activation assay with protein phosphatase 5,
wherein said phosphatase activation assay comprises
- measuring the enzymatic activity of protein phosphatase 5 in the absence of a compound to be tested, and
- measuring the enzymatic activity of protein phosphatase 5 under the same conditions, but in the presence of said compound to be tested,
and wherein a result in said phosphatase activation assay is considered a positive result if the enzymatic activity of protein phosphatase 5 in the presence of the compound to be tested is by at least a factor of 1.5 higher than in the absence of the compound to be tested,
- and, preferably, determining in an *in vitro* binding assay if said compound binds to protein phosphatase 5 within the region of amino acids 166 to 496 of the protein phosphatase 5 sequence,
wherein said compound is considered a successful candidate in said screening method if it shows a positive result in said phosphatase activation assay and, preferably, if it binds to protein phosphatase 5 within the region of amino acids 166 to 496 of the protein phosphatase 5 sequence in said *in vitro* binding assay.

5. The compound according to any of claims 1-3, wherein said protein phosphatase 5 is protein phosphatase 5 from *C*. *elegans,* rat protein phosphatase 5, mouse protein phosphatase 5, or human protein phosphatase 5.

6. The compound according to any of claims 2, 3 or 5, wherein said *in vitro* binding assay is NMR measurements.

7. The compound according to any of claims 1, 3 or 5-6, wherein said compound does not show a positive result in a phosphatase activation assay with the phosphatase PP1, PP2A, PP2B/PP3, or alkaline phosphatase.

8. The compound according to any of claims 1-3 or 5-7, wherein said compound does not bind to the TPR (tetratricopeptide repeat) domain of protein phosphatase 5.

9. The compound according to any of claims 1-3 or 5-8, wherein said compound is for use in the treatment of a tauopathy,
preferably a tauopathy selected from the group consisting of Alzheimer's disease, amyotrophic lateral sclerosis and parkinsonism-dementia complex, argyrophilic grain disease, chronic traumatic encephalopathy, chronic traumatic encephalopathy, traumatic brain injury, diffuse neurofibrillary tangles with calcification, Down's syndrome, familial British dementia, familial Danish dementia, frontotemporal dementia and parkinsonism linked to chromosome 17 (caused by MAPT mutations), frontotemporal lobar degeneration, preferably caused by C9ORF72 mutations, Gerstmann-Sträussler-Scheinker disease, Guadeloupean parkinsonism, myotonic dystrophy, neurodegeneration with brain iron accumulation, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, SLC9A6-related mental retardation, subacute sclerosing panencephalitis, tangle-only dementia and white matter tauopathy with globular glial inclusions,
more preferably a tauopathy selected from the group consisting of Alzheimer's disease, chronic traumatic encephalopathy, frontotemporal dementia, parkinsonism linked to chromosome 17 (caused by MAPT mutations), frontotemporal lobar degeneration, preferably caused by C9ORF72 mutations, Niemann-Pick disease type C, Pick's disease, progressive supranuclear palsy, tangle-only dementia, and white matter tauopathy with globular glial inclusions,
or for use in the treatment of cancer,
preferably a cancer of the blood, bile duct, bladder, bone, brain, breast, cervix, colon, esophagus, eye, gallbladder, germ cells, intestines, kidney larynx, lip, liver, lung, mouth, nose, oral cavity, ovaries, pancreas, penis, pharynx, prostate, skin, stomach, testicles, throat, thymus, thyroid, urethra, or vagina,
more preferably a cancer selected from the group consisting of acute lymphoblastic leukemia (ALL), acute myeloid leukemia, astrocytoma, basal-cell carcinoma, bile duct cancer, bladder cancer, bone tumor, brain tumor, breast cancer, Burkitt's lymphoma, cervical cancer, chronic myelogenous leukemia (CML), colon cancer, emphysema, esophageal cancer, intraocular melanoma, eye cancer, retinoblastoma, gallbladder cancer, gastric (stomach) cancer, gastrointestinal stromal tumor (GIST), germ cell tumor, glioma, head and neck cancer, hepatocellular (liver) cancer, Hodgkin lymphoma, islet cell carcinoma (endocrine pancreas), Kaposi sarcoma, kidney cancer, renal cell cancer, laryngeal cancer, leukemia, acute lymphoblastic leukemia, acute myelogenous leukemia, lip and oral cavity cancer, liver cancer, non-small cell lung cancer, small cell lung cancer, lymphoma, preferably Burkitt lymphoma, Hodgkin lymphoma or Non-Hodgkin lymphoma, medulloblastoma, melanoma, mesothelioma, mouth cancer, plasma cell neoplasm, myelodysplastic/myeloproliferative diseases, multiple myeloma, nasal cavity and paranasal sinus cancer, nasopharyngeal carcinoma, neuroblastoma, oral cancer, oropharyngeal cancer, osteosarcoma, ovarian cancer, pancreatic cancer, pancreatic carcinoma, pancreatic islet cell cancer, parathyroid cancer, penile cancer, pharyngeal cancer, prostate cancer, rectal cancer, renal cell carcinoma, salivary gland cancer, sarcoma, soft tissue sarcoma, stomach cancer, T-cell lymphoma, Sézary syndrome, testicular cancer, throat cancer, thymoma, thymic carcinoma, thyroid cancer, urethral cancer, uterine cancer, vaginal cancer, vulvar cancer, Waldenström macroglobulinemia, and Wilms tumor,
most preferably pancreatic carcinoma.

10. The compound according to any of claims 1-3 or 5-9, wherein
said compound has a general structure as defined in any of the following **(i)-(v):**
(i) said compound has the general structure
*A-B*,
wherein *A* is
- unsubstituted phenyl or
- phenyl substituted with one or more substituents,
wherein the one or more substituents are independently selected from the group consisting of
- halogen (preferably F, Cl, Br, or I),
- hydroxyl,
- amino,
- nitro,
- cyano,
- thiol,
- sulfonyl,
- carbonyl, preferably an aldehyde, ketone, ester or amide,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- substituted or unsubstituted cycloalkyl, and
- substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms, wherein, preferably, one of the substituents is Cl in para-position to *B*,
wherein, preferably, *A* is phenyl substituted with one or more substituents, preferably with one substituent,
wherein, preferably, at least one substituent is in para-position to *B*, wherein, preferably, the one or more substituents are independently selected from the group consisting of F, Cl, and Br, wherein, more preferably, the one or more substituents are all Cl,
wherein, most preferably, *A* is 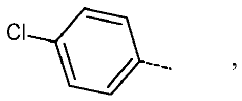
and wherein *B* is 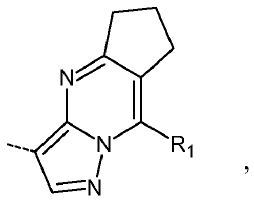 wherein R₁ is 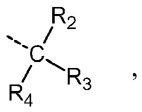 wherein R₂, R₃ and R₄ are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
wherein, preferably, each of R₂, R₃ and R₄ comprises up to 14, preferably up to 10, more preferably up to 6, more preferably up to 3, more preferably up to 2, more preferably up to 1 carbon atoms,
wherein, preferably, *B* is 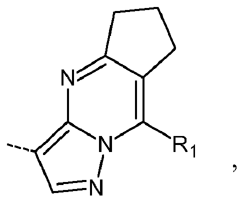 wherein R₁ is 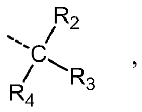 wherein R₂, R₃ and R₄ are independently selected from the group consisting of F, Cl, and Br, wherein, preferably, at least one of R₂, R₃ and R₄, more preferably at least two of R₂, R₃ and R₄ are F,
wherein, more preferably, *B* is 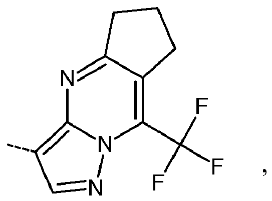 wherein, most preferably, said compound has the structure represented by Formula I: 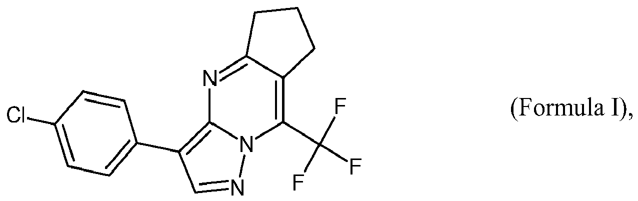 or
**(ii)** said compound has the general structure
*A-B-C-D*,
wherein *A* is
- unsubstituted phenyl or
- phenyl substituted with one or more substituents,
wherein the one or more substituents are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen,
hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- substituted or unsubstituted cycloalkyl, and
- substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is Cl in para-position to *B*,
wherein, preferably, *A* is phenyl substituted with one or more substituents, preferably with one substituent,
wherein, preferably, at least one substituent is in para-position to *B*, wherein, preferably, the one or more substituents are independently selected from the group consisting of F, Cl, and Br, wherein, more preferably, the one or more substituents are all Cl,
wherein, most preferably, A is 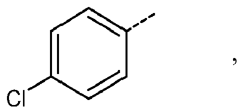
wherein *B* is 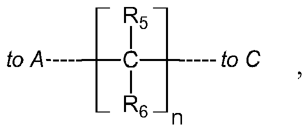 wherein n is from 1 to 5, preferably from 1 to 4, more preferably from 1 to 3, more preferably 1 or 2, more preferably 1,
and R₅ and R₆ are selected, independently for each occurrence of R₅ resp. R₆, from the group consisting of hydrogen and methyl,
wherein, preferably, *B* is 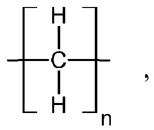 wherein n is from 1 to 3, preferably 1 or 2, more preferably 1,
wherein, most preferably, *B* is 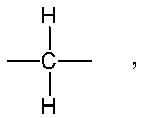 wherein *C* is 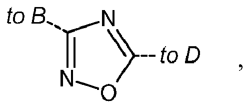 and wherein *D* is
- unsubstituted phenyl or
- phenyl substituted with one or more substituents, wherein the one or more substituents are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- substituted or unsubstituted cycloalkyl, and
- substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one substituent is a methoxy group in meso-position to Cand one substituent is a methoxy group in para-position to *C*,
wherein, preferably, *D* is phenyl substituted with one or more substituents, more preferably substituted with two substituents,
wherein, preferably, one substituent is in meso-position to *C* and one in para-position to *C*,
wherein, preferably, the one or more substituents are independently selected from the group consisting of straight alkoxy groups,
wherein, preferably, each of the one or more substituents comprises up to 3, preferably up to 2, more preferably up to 1 carbon atoms,
wherein, most preferably, *D* is 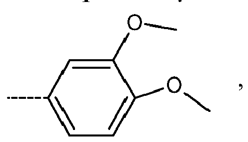
wherein, most preferably, said compound has the structure represented by Formula II: 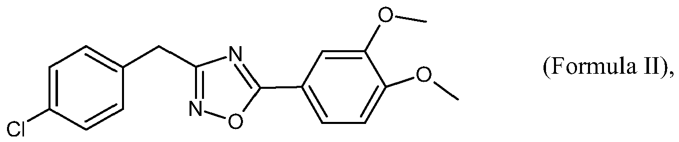 or
(iii) said compound said compound has the general structure
*A-B-C-D*,
wherein *A* comprises a non-planar group comprising 6 to 13 carbon atoms, preferably 8 to 12 carbon atoms, more preferably 9-11 carbon atoms, more preferably 10 carbon atoms and has, preferably, a molecular weight of up to 300 Dalton,
wherein, preferably, *A* is a triyclic group,
wherein, preferably, *A* is composed exclusively of carbon and hydrogen atoms,
wherein, most preferably, *A* is 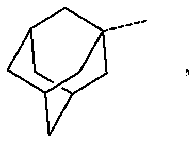 wherein *B* is 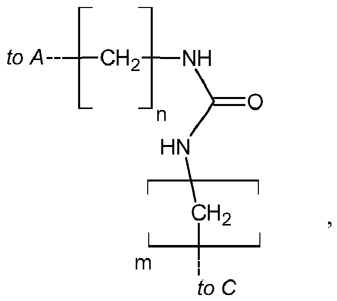 wherein n is from 0 to 4, preferably 0 or 1,
and wherein m is from 0 to 4, preferably from 0 to 2, more preferably 1 or 2, more preferably 1,
wherein, preferably, *B* is 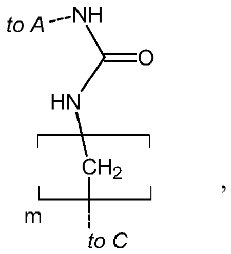 wherein m is from 0 to 4, preferably from 0 to 2, more preferably 1 or 2,
wherein, most preferably, *B* is 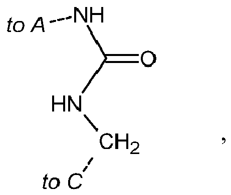 wherein C is 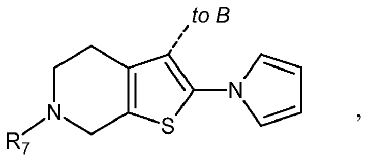 wherein R₇ is a straight or branched alkyl chain with up to 4 carbon atoms, preferably an alkyl chain with up to 2 carbon atoms, most preferably a methyl group,
wherein, most preferably, said compound has the structure represented by Formula III: 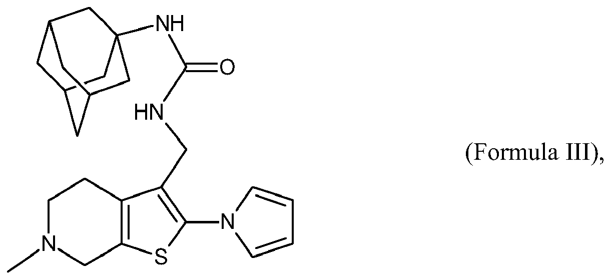 or
(iv) said compound has the general structure
*A-B-C-D*,
wherein *A* is
- unsubstituted phenyl or
- phenyl substituted with one or more substituents,
wherein the one or more substituents are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- substituted or unsubstituted cycloalkyl, and
- substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is F in para-position to *B*,
wherein, preferably, *A* is phenyl substituted with one or more substituents, preferably with one substituent,
wherein, preferably, at least one substituent is in para-position to *B*, wherein, preferably, the one or more substituents are independently selected from the group consisting of F, Cl, and Br, wherein, more preferably, the one or more substituents are all F,
wherein, most preferably, *A* is 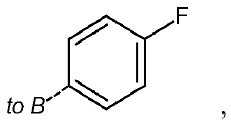 wherein *B* is 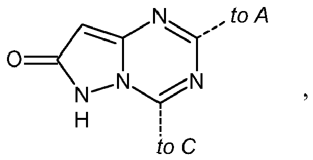 wherein *C* is 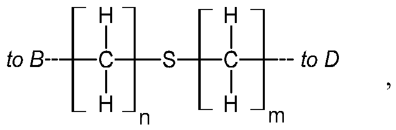 wherein n is from 0 to 4, preferably 0 or 1,
and wherein m is from 0 to 4, preferably from 0 to 2,
wherein, preferably, *C* is 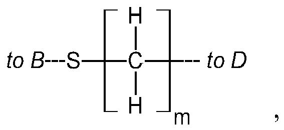 wherein m is from 0 to 4, preferably from 0 to 2, more preferably 1 or 2,
wherein, most preferably, *C* is 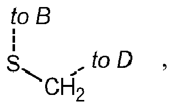 and wherein *D* is
- unsubstituted phenyl or
- phenyl substituted with one or more substituents, wherein the one or more substituents are independently selected from the group consisting of
- halogen (preferably F, Cl, Br, or I),
- hydroxyl,
- amino,
- nitro,
- cyano,
- thiol,
- sulfonyl,
- carbonyl,
- carboxyl,
- straight or branched alkyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkoxy, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- straight or branched alkenyl, which is unsubstituted or substituted, preferably with one or more substituents selected from the group consisting of halogen, hydroxyl, amino, nitro, cyano, thiol, sulfonyl, carbonyl, carboxyl, alkyl, alkoxy, alkenyl, cycloalkyl and aryl,
- substituted or unsubstituted cycloalkyl, and
- substituted or unsubstituted aryl, preferably substituted or unsubstituted phenyl,
wherein, preferably, each of the one or more substituents comprises up to 18, preferably up to 14, more preferably up to 10, more preferably up to 6, more preferably up to 5, more preferably up to 4, more preferably up to 3, more preferably up to 2, more preferably up to 1 carbon atoms,
wherein, preferably, one of the substituents is Br in para-position to C,
wherein, preferably, *D* is phenyl substituted with one or more substituents, preferably with one substituent,
wherein, preferably, at least one substituent is in para-position to *C*, wherein, preferably, the one or more substituents are independently selected from the group consisting of F, Cl, and Br, wherein, more preferably, the one or more substituents are all Br,
wherein, most preferably, *D* is 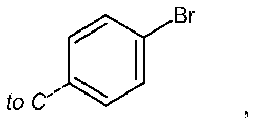 wherein, most preferably, said compound has the structure represented by Formula IV: 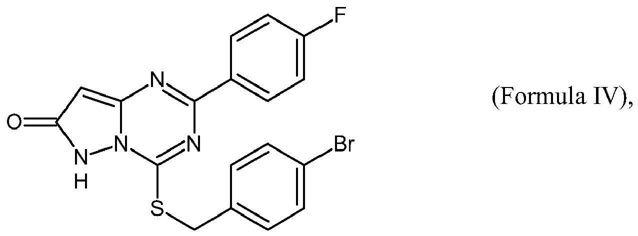 or
(v) said compound has the general structure *A-B-C-D-C-B-A*
or
*A-B-C-D-*R₉,
wherein *A* is 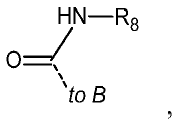 wherein R₈ is a straight or branched alkyl chain with up to 4 carbon atoms, preferably an alkyl chain with up to 2 carbon atoms, most preferably a methyl group,
wherein *B* is 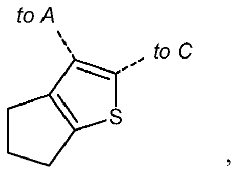 wherein *C* is 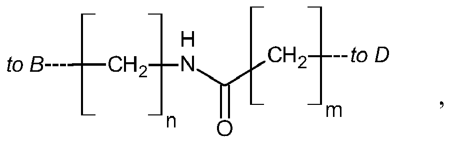 wherein n is from 0 to 4, preferably 0 or 1,
and wherein m is from 0 to 4, preferably from 0 to 2,
wherein, preferably, *C* is 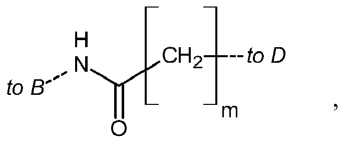 wherein m is from 0 to 4, preferably from 0 to 2, more preferably 1 or 2,
wherein, most preferably, *C* is 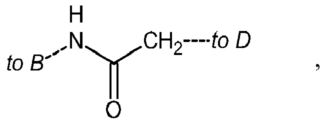 and wherein *D* is 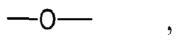 wherein A is the same or different for each occurrence,
wherein B is the same or different for each occurrence,
wherein C is the same or different for each occurrence,
wherein R₉ is hydrogen, methyl or straight or branched alkyl which is unsubstituted or substituted with one or more substituents selected from the group consisting of halogen, preferably F, Cl, Br, or I, hydroxyl, amino, nitro, cyano, thiol, sulfonyl, carbonyl, carboxyl, substituted or unsubstituted aryl, preferably substituted or unsubstituted phenyl, and substituted or unsubstituted heteroaryl,
wherein, preferably, the molecular weight of R₉ is below 2000 Dalton, more preferably below 1000 Dalton, more preferably below 500 Dalton, more preferably below 450 Dalton, more preferably below 250 Dalton, more preferably below 200 Dalton, more preferably below 150 Dalton, more preferably below 100 Dalton, more preferably below 50 Dalton,
wherein, most preferably, said compound has the structure represented by Formula V:

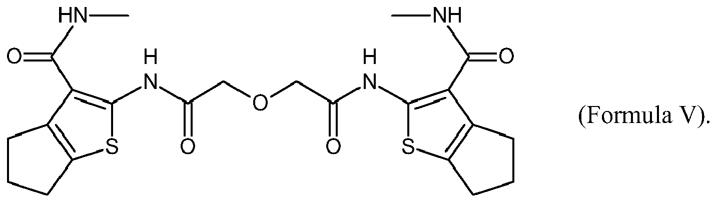

11. A method for activating protein phosphatase 5, said method comprising the steps of contacting protein phosphatase 5 with a compound according to any of claims 1-3 or 5-10 in an amount sufficient to activate protein phosphatase 5.

12. A pharmaceutical composition comprising one or more compounds as defined in any of claims 1-3 or 5-11.


SEQUENCE LISTING

<110>  Deutsches Zentrum für Neurodegenerative Erkrankungen / TU München

<120>  Activators of Protein Phosphatase 5

<130>  D31190EP

<160>  4

<170>  PatentIn version 3.5

<210>  1
<211>  496
<212>  PRT
<213>  Caenorhabditis elegans

<400>  1

Met Ala Ala Thr Ile Thr Asp Asp Ile Val Ala Thr Val Leu Glu Ser
1               5                   10                  15

Ile Glu Glu Lys Ser Tyr Glu Asp Glu Lys Glu Lys Ala Gly Met Ile
            20                  25                  30

Lys Asp Glu Ala Asn Gln Phe Phe Lys Asp Gln Val Tyr Asp Val Ala
        35                  40                  45

Ala Asp Leu Tyr Ser Val Ala Ile Glu Ile His Pro Thr Ala Val Leu
    50                  55                  60

Tyr Gly Asn Arg Ala Gln Ala Tyr Leu Lys Lys Glu Leu Tyr Gly Ser
65                  70                  75                  80

Ala Leu Glu Asp Ala Asp Asn Ala Ile Ala Ile Asp Pro Ser Tyr Val
                85                  90                  95

Lys Gly Phe Tyr Arg Arg Ala Thr Ala Asn Met Ala Leu Gly Arg Phe
            100                 105                 110

Lys Lys Ala Leu Thr Asp Tyr Gln Ala Val Val Lys Val Cys Pro Asn
        115                 120                 125

Asp Lys Asp Ala Arg Ala Lys Phe Asp Glu Cys Ser Lys Ile Val Arg
    130                 135                 140

Arg Gln Lys Phe Glu Ala Ala Ile Ser Thr Asp His Asp Lys Lys Thr
145                 150                 155                 160

Val Ala Glu Thr Leu Asp Ile Asn Ala Met Ala Ile Glu Asp Ser Tyr
                165                 170                 175

Asp Gly Pro Arg Leu Glu Asp Lys Ile Thr Lys Glu Phe Val Leu Gln
        180                 185                 190

Leu Ile Lys Thr Phe Lys Asn Gln Gln Lys Leu His Lys Lys Tyr Ala
    195                 200                 205

Phe Lys Met Leu Leu Glu Phe Tyr Asn Tyr Val Lys Ser Leu Pro Thr
    210                 215                 220

Met Val Glu Ile Thr Val Pro Thr Gly Lys Lys Phe Thr Ile Cys Gly
225                 230                 235                 240

Asp Val His Gly Gln Phe Tyr Asp Leu Cys Asn Ile Phe Glu Ile Asn
                245                 250                 255

Gly Tyr Pro Ser Glu Thr Asn Pro Tyr Leu Phe Asn Gly Asp Phe Val
            260                 265                 270

Asp Arg Gly Ser Phe Ser Val Glu Thr Ile Phe Thr Met Ile Gly Phe
        275                 280                 285

Lys Leu Leu Tyr Pro Asn His Phe Phe Met Ser Arg Gly Asn His Glu
    290                 295                 300

Ser Asp Val Met Asn Lys Met Tyr Gly Phe Glu Gly Glu Val Lys Ala
305                 310                 315                 320

Lys Tyr Thr Gln Gln Met Cys Asp Met Phe Thr Glu Thr Phe Cys Trp
                325                 330                 335

Leu Pro Leu Cys His Leu Ile Asn Glu Lys Ile Phe Val Cys His Gly
            340                 345                 350

Gly Leu Phe Lys Glu Asp Gly Val Thr Leu Glu Asp Ile Arg Lys Thr
        355                 360                 365

Asp Arg Asn Arg Gln Pro Pro Asp Glu Gly Ile Met Cys Asp Leu Leu
    370                 375                 380

Trp Ser Asp Pro Gln Pro Ile Asn Gly Arg Ser Pro Ser Lys Arg Gly
385                 390                 395                 400

Val Gly Cys Gln Phe Gly Pro Asp Val Thr Ser Lys Trp Cys Glu Thr
                405                 410                 415

Asn Gly Ile Glu Tyr Val Val Arg Ser His Glu Val Lys Pro Glu Gly
            420                 425                 430

Tyr Glu Met His His Asn Gly Gln Cys Phe Thr Val Phe Ser Ala Pro
        435                 440                 445

Asn Tyr Cys Asp Gln Met Asn Asn Lys Gly Ala Phe Ile Thr Ile Thr
    450                 455                 460

Gly Asp Asn Leu Thr Pro Arg Phe Thr Pro Phe Asp Ala Val Pro His
465                 470                 475                 480

Pro Lys Leu Pro Pro Met Ala Tyr Ala Asn Ser Leu Phe Gly Phe Asn
                485                 490                 495

<210>  2
<211>  499
<212>  PRT
<213>  Rattus norvegicus

<400>  2

Met Ala Met Ala Glu Gly Glu Arg Thr Glu Cys Ala Glu Pro Pro Arg
1               5                   10                  15

Asp Glu Pro Pro Ala Glu Gly Thr Leu Lys Arg Ala Glu Glu Leu Lys
            20                  25                  30

Thr Gln Ala Asn Asp Tyr Phe Lys Ala Lys Asp Tyr Glu Asn Ala Ile
        35                  40                  45

Lys Phe Tyr Ser Gln Ala Ile Glu Leu Asn Pro Ser Asn Ala Ile Tyr
    50                  55                  60

Tyr Gly Asn Arg Ser Leu Ala Tyr Leu Arg Thr Glu Cys Tyr Gly Tyr
65                  70                  75                  80

Ala Leu Gly Asp Ala Thr Arg Ala Ile Glu Leu Asp Lys Lys Tyr Ile
                85                  90                  95

Lys Gly Tyr Tyr Arg Arg Ala Ala Ser Asn Met Ala Leu Gly Lys Phe
            100                 105                 110

Arg Ala Ala Leu Arg Asp Tyr Glu Thr Val Val Lys Val Lys Pro Asn
        115                 120                 125

Asp Lys Asp Ala Lys Met Lys Tyr Gln Glu Cys Ser Lys Ile Val Lys
    130                 135                 140

Gln Lys Ala Phe Glu Arg Ala Ile Ala Gly Asp Glu His Arg Arg Ser
145                 150                 155                 160

Val Val Asp Ser Leu Asp Ile Glu Ser Met Thr Ile Glu Asp Glu Tyr
                165                 170                 175

Ser Gly Pro Lys Leu Glu Asp Gly Lys Val Thr Ile Thr Phe Met Lys
            180                 185                 190

Asp Leu Met Gln Trp Tyr Lys Asp Gln Lys Lys Leu His Arg Lys Cys
        195                 200                 205

Ala Tyr Gln Ile Leu Val Gln Val Lys Glu Val Leu Cys Lys Leu Ser
    210                 215                 220

Thr Leu Val Glu Thr Thr Leu Lys Glu Thr Glu Lys Ile Thr Val Cys
225                 230                 235                 240

Gly Asp Thr His Gly Gln Phe Tyr Asp Leu Leu Asn Ile Phe Glu Leu
                245                 250                 255

Asn Gly Leu Pro Ser Glu Thr Asn Pro Tyr Ile Phe Asn Gly Asp Phe
            260                 265                 270

Val Asp Arg Gly Ser Phe Ser Val Glu Val Ile Leu Thr Leu Phe Gly
        275                 280                 285

Phe Lys Leu Leu Tyr Pro Asp His Phe His Leu Leu Arg Gly Asn His
    290                 295                 300

Glu Thr Asp Asn Met Asn Gln Ile Tyr Gly Phe Glu Gly Glu Val Lys
305                 310                 315                 320

Ala Lys Tyr Thr Ala Gln Met Tyr Glu Leu Phe Ser Glu Val Phe Glu
                325                 330                 335

Trp Leu Pro Leu Ala Gln Cys Ile Asn Gly Lys Val Leu Ile Met His
            340                 345                 350

Gly Gly Leu Phe Ser Glu Asp Gly Val Thr Leu Asp Asp Ile Arg Lys
        355                 360                 365

Ile Glu Arg Asn Arg Gln Pro Pro Asp Ser Gly Pro Met Cys Asp Leu
    370                 375                 380

Leu Trp Ser Asp Pro Gln Pro Gln Asn Gly Arg Ser Val Ser Lys Arg
385                 390                 395                 400

Gly Val Ser Cys Gln Phe Gly Pro Asp Val Thr Lys Ala Phe Leu Glu
                405                 410                 415

Glu Asn Gln Leu Asp Tyr Ile Ile Arg Ser His Glu Val Lys Ala Glu
            420                 425                 430

Gly Tyr Glu Val Ala His Gly Gly Arg Cys Val Thr Val Phe Ser Ala
        435                 440                 445

Pro Asn Tyr Cys Asp Gln Met Gly Asn Lys Ala Ser Tyr Ile His Leu
    450                 455                 460

Gln Gly Ser Asp Leu Arg Pro Gln Phe His Gln Phe Thr Ala Val Pro
465                 470                 475                 480

His Pro Asn Val Lys Pro Met Ala Tyr Ala Asn Thr Leu Leu Gln Leu
                485                 490                 495

Gly Met Met

<210> 3
<211> 499
<212> PRT
<213> Mus musculus

<400> 3

Met Ala Met Ala Glu Gly Glu Arg Thr Glu Cys Ala Glu Thr Pro Arg
1               5                   10                  15

Asp Glu Pro Pro Ala Asp Gly Ala Leu Lys Arg Ala Glu Glu Leu Lys
            20                  25                  30

Thr Gln Ala Asn Asp Tyr Phe Lys Ala Lys Asp Tyr Glu Asn Ala Ile
        35                  40                  45

Lys Phe Tyr Ser Gln Ala Ile Glu Leu Asn Pro Gly Asn Ala Ile Tyr
    50                  55                  60

Tyr Gly Asn Arg Ser Leu Ala Tyr Leu Arg Thr Glu Cys Tyr Gly Tyr
65                  70                  75                  80

Ala Leu Gly Asp Ala Thr Arg Ala Ile Glu Leu Asp Lys Lys Tyr Ile
                85                  90                  95

Lys Gly Tyr Tyr Arg Arg Ala Ala Ser Asn Met Ala Leu Gly Lys Phe
            100                 105                 110

Arg Ala Ala Leu Arg Asp Tyr Glu Thr Val Val Lys Val Lys Pro Asn
        115                 120                 125

Asp Lys Asp Ala Lys Met Lys Tyr Gln Glu Cys Ser Lys Ile Val Lys
    130                 135                 140

Gln Lys Ala Phe Glu Arg Ala Ile Ala Gly Asp Glu His Arg Arg Ser
145                 150                 155                 160

Val Val Asp Ser Leu Asp Ile Glu Ser Met Thr Ile Glu Asp Glu Tyr
                165                 170                 175

Ser Gly Pro Lys Leu Glu Asp Gly Lys Val Thr Ile Thr Phe Met Lys
            180                 185                 190

Asp Leu Met Gln Trp Tyr Lys Asp Gln Lys Lys Leu His Arg Lys Cys
        195                 200                 205

Ala Tyr Gln Ile Leu Val Gln Val Lys Glu Val Leu Cys Lys Leu Ser
    210                 215                 220

Thr Leu Val Glu Thr Thr Leu Lys Glu Thr Glu Lys Ile Thr Val Cys
225                 230                 235                 240

Gly Asp Thr His Gly Gln Phe Tyr Asp Leu Leu Asn Ile Phe Glu Leu
                245                 250                 255

Asn Gly Leu Pro Ser Glu Thr Asn Pro Tyr Ile Phe Asn Gly Asp Phe
            260                 265                 270

Val Asp Arg Gly Ser Phe Ser Val Glu Val Ile Leu Thr Leu Phe Gly
        275                 280                 285

Phe Lys Leu Leu Tyr Pro Asp His Phe His Leu Leu Arg Gly Asn His
    290                 295                 300

Glu Thr Asp Asn Met Asn Gln Ile Tyr Gly Phe Glu Gly Glu Val Lys
305                 310                 315                 320

Ala Lys Tyr Thr Ala Gln Met Tyr Glu Leu Phe Ser Glu Val Phe Glu
                325                 330                 335

Trp Leu Pro Leu Ala Gln Cys Ile Asn Gly Lys Val Leu Ile Met His
            340                 345                 350

Gly Gly Leu Phe Ser Glu Asp Gly Val Thr Leu Asp Asp Ile Arg Lys
        355                 360                 365

Ile Glu Arg Asn Arg Gln Pro Pro Asp Ser Gly Pro Met Cys Asp Leu

370                 375                 380

Leu Trp Ser Asp Pro Gln Pro Gln Asn Gly Arg Ser Val Ser Lys Arg
385                 390                 395                 400

Gly Val Ser Cys Gln Phe Gly Pro Asp Val Thr Lys Ala Phe Leu Glu
                405                 410                 415

Glu Asn Gln Leu Asp Tyr Ile Ile Arg Ser His Glu Val Lys Ala Glu
            420                 425                 430

Gly Tyr Glu Val Ala His Gly Gly Arg Cys Val Thr Val Phe Ser Ala
        435                 440                 445

Pro Asn Tyr Cys Asp Gln Met Gly Asn Lys Ala Ser Tyr Ile His Leu
    450                 455                 460

Gln Gly Ser Asp Leu Arg Pro Gln Phe His Gln Phe Thr Ala Val Pro
465                 470                 475                 480

His Pro Asn Val Lys Pro Met Ala Tyr Ala Asn Thr Leu Leu Gln Leu
                485                 490                 495

Gly Met Met

<210>  4
<211>  499
<212>  PRT
<213>  Homo sapiens

<400>  4

Met Ala Met Ala Glu Gly Glu Arg Thr Glu Cys Ala Glu Pro Pro Arg
1               5                   10                  15

Asp Glu Pro Pro Ala Asp Gly Ala Leu Lys Arg Ala Glu Glu Leu Lys
            20                  25                  30

Thr Gln Ala Asn Asp Tyr Phe Lys Ala Lys Asp Tyr Glu Asn Ala Ile
        35                  40                  45

Lys Phe Tyr Ser Gln Ala Ile Glu Leu Asn Pro Ser Asn Ala Ile Tyr
    50                  55                  60

Tyr Gly Asn Arg Ser Leu Ala Tyr Leu Arg Thr Glu Cys Tyr Gly Tyr
65                  70                  75                  80

Ala Leu Gly Asp Ala Thr Arg Ala Ile Glu Leu Asp Lys Lys Tyr Ile

85                  90                  95

Lys Gly Tyr Tyr Arg Arg Ala Ala Ser Asn Met Ala Leu Gly Lys Phe
            100                 105                 110

Arg Ala Ala Leu Arg Asp Tyr Glu Thr Val Val Lys Val Lys Pro His
        115                 120                 125

Asp Lys Asp Ala Lys Met Lys Tyr Gln Glu Cys Asn Lys Ile Val Lys
    130                 135                 140

Gln Lys Ala Phe Glu Arg Ala Ile Ala Gly Asp Glu His Lys Arg Ser
145                 150                 155                 160

Val Val Asp Ser Leu Asp Ile Glu Ser Met Thr Ile Glu Asp Glu Tyr
                165                 170                 175

Ser Gly Pro Lys Leu Glu Asp Gly Lys Val Thr Ile Ser Phe Met Lys
            180                 185                 190

Glu Leu Met Gln Trp Tyr Lys Asp Gln Lys Lys Leu His Arg Lys Cys
        195                 200                 205

Ala Tyr Gln Ile Leu Val Gln Val Lys Glu Val Leu Ser Lys Leu Ser
    210                 215                 220

Thr Leu Val Glu Thr Thr Leu Lys Glu Thr Glu Lys Ile Thr Val Cys
225                 230                 235                 240

Gly Asp Thr His Gly Gln Phe Tyr Asp Leu Leu Asn Ile Phe Glu Leu
                245                 250                 255

Asn Gly Leu Pro Ser Glu Thr Asn Pro Tyr Ile Phe Asn Gly Asp Phe
            260                 265                 270

Val Asp Arg Gly Ser Phe Ser Val Glu Val Ile Leu Thr Leu Phe Gly
        275                 280                 285

Phe Lys Leu Leu Tyr Pro Asp His Phe His Leu Leu Arg Gly Asn His
    290                 295                 300

Glu Thr Asp Asn Met Asn Gln Ile Tyr Gly Phe Glu Gly Glu Val Lys
305                 310                 315                 320

Ala Lys Tyr Thr Ala Gln Met Tyr Glu Leu Phe Ser Glu Val Phe Glu
                325                 330                 335

Trp Leu Pro Leu Ala Gln Cys Ile Asn Gly Lys Val Leu Ile Met His
            340                 345                 350

Gly Gly Leu Phe Ser Glu Asp Gly Val Thr Leu Asp Asp Ile Arg Lys
        355                 360                 365

Ile Glu Arg Asn Arg Gln Pro Pro Asp Ser Gly Pro Met Cys Asp Leu
    370                 375                 380

Leu Trp Ser Asp Pro Gln Pro Gln Asn Gly Arg Ser Ile Ser Lys Arg
385                 390                 395                 400

Gly Val Ser Cys Gln Phe Gly Pro Asp Val Thr Lys Ala Phe Leu Glu
                405                 410                 415

Glu Asn Asn Leu Asp Tyr Ile Ile Arg Ser His Glu Val Lys Ala Glu
            420                 425                 430

Gly Tyr Glu Val Ala His Gly Gly Arg Cys Val Thr Val Phe Ser Ala
        435                 440                 445

Pro Asn Tyr Cys Asp Gln Met Gly Asn Lys Ala Ser Tyr Ile His Leu
    450                 455                 460

Gln Gly Ser Asp Leu Arg Pro Gln Phe His Gln Phe Thr Ala Val Pro
465                 470                 475                 480

His Pro Asn Val Lys Pro Met Ala Tyr Ala Asn Thr Leu Leu Gln Leu
                485                 490                 495

Gly Met Met

2

1

1